# EUROPEAN PATENT SPECIFICATION

(11) **EP 1 242 629 B1**
(45) Date of publication and mention of the grant of the patent: **26.10.2005**
(21) Application number: 00991290.8
(22) Date of filing: 28.12.2000
(51) Int. Cl.: C12Q 1/68, C12N 5/00, C12N 15/00, A01K 67/027

(54) **IMPLICATION OF A KNOWN GENE NAMED CP2/LSF-LBP-1 IN ALZHEIMER'S DESEASE**
ZUSAMMENHANG DES BEKANNTEN GENS CP2/LSF/LBP-1 MIT DER ALZHEIMERSCHEN KRANKHEIT
IMPLICATION D'UN GENE CONNU APPELE CP2/LSF-LBP-1 DANS LA MALADIE D'ALZHEIMER

(30) Priority: 28.12.1999 EP 99403304
(43) Date of publication of application: 25.09.2002
(73) Proprietor: INSTITUT PASTEUR DE LILLE, 59019 Lille cedex (FR); INSTITUT NATIONAL DE LA SANTE ET DE LA RECHERCHE MEDICALE (INSERM), 75013 Paris (FR)
(72) Inventor: CHARTIER-HARLIN, Marie-Christine, F-59139 Wattignies (FR); AMOUYEL, Philippe, F-59700 Marcq-en-Baroeul (FR); LAMBERT, Jean-Charles Dep. of Psych. Div. Neurosc., Birmingham B15 2QZ (GB); ARARIA, Louise, F-59100 Roubais (FR)
(74) Representative: Bernasconi, Jean Raymond
(86) International application number: PCT/EP2000/013408
(87) International publication number: WO 2001/048240

(56) References cited:
- WO-A-95/29257
- WO-A-98/36641
- FR-A- 2 765 591
- SWENDEMAN ET AL.: "CHARACTERIZATION OF THE GENOMIC STRUCTURE, CHROMOSOMAL LOCATION, PROMOTER, AND DEVELOPMENTAL EXPRESSION OF THE ALPHA-GLOBIN TRANSCRIPTION FACTOR CP2" J.BIOL.CHEM., vol. 269, no. 15, 1994, pages 11663-11671, XP002142009 cited in the application
- PERICAK-VANCE ET AL.: "COMPLETE GENOMIC SCREEN IN LATE-ONSET FAMILIAL ALZHEIMER DISEASE" JAMA, vol. 278, no. 15, 1997, pages 1237-1241, XP000915059 cited in the application & DATABASE NCBI [Online] GENOME, HOMO SAPIENS MAP VIEW, D12S390, "CHROMOSOME 12: 43.42-45.02 cR"
- BING ET AL.: "PURIFICATION AND CHARACTERIZATION OF THE SERUM AMYLOID A3 ENHANCER FACTOR" J.BIOL.CHEM., vol. 274, no. 35, August 1999 (1999-08), pages 24649-24656, XP002142011 cited in the application
- RUSSO ET AL.: "Fe65 AND THE PROTEIN NETWORK CENTERED AROUND THE CYTOSOLIC DOMAIN OF THE ALZHEIMER'S BETA-AMYLOID PRECURSOR PROTEIN" FEBS LETTERS, vol. 434, 1998, pages 1-7, XP002142012 cited in the application
- ZAMBRANO ET AL.: "THE Fe65 ADAPTOR PROTEIN INTERACTS THROUGH ITS PID1 DOMAIN WITH THE TRANSCRIPTION FACTOR CP2/LSF/LBP1" J.BIOL.CHEM., vol. 273, no. 32, 1998, pages 20128-20133, XP002142013
- LAU KWOK-FAI ET AL: "Molecular cloning and characterization of the human glycogen synthase kinase-3beta promoter." GENOMICS, vol. 60, no. 2, pages 121-128, XP002175133 ISSN: 0888-7543
- DATABASE EMBL [Online] ID:AC011600, 11 October 1999 (1999-10-11) MUZNY D.M. ET AL.: "Homo sapiens chromosome 12 clone RP11-392I16" XP002175135
- LAMBERT JEAN-CHARLES ET AL: "The transcriptional factor LBP-1c/CP2/LSF gene on chromosome 12 is a genetic determinant of Alzheimer's disease." HUMAN MOLECULAR GENETICS, vol. 9, no. 15, 2000, pages 2275-2280, XP002175134 ISSN: 0964-6906

## Description

The invention relates to the implication of a known gene named CP2/LSF-LBP-1 in Alzheimer's disease, namely in sporadic forms of Alzheimer's disease.

Alzheimer's disease is a neurodegenerative process characterised by two main cerebral lesions : amyloid plaques and neurofibrillary degeneration. Numerous data suggested that the first step of the development of the disease would be the formation of the senile plaques, leading to neurodegeneration and finally to dementia. To better understand the etiology of the pathology, a systematic search for genes responsible of the monogenic familial forms of Alzheimer's disease have been undertaken. At present, three genes have been discovered, bearing pathogenic mutations : the APP gene on chromosome 21 (Goate et al, 1991), the PS1 and PS2 genes on chromosome 14 and 1 respectively (Sherrington et al, 1995 ; Levy-Lahad et al, 1995).

Even if the discovery of these mutations were essential for the understanding of the Alzheimer's disease process, they explain only less than 2% of all Alzheimer's disease cases (Cruts et al, 1998). Indeed, most of the forms are sporadic, without obvious mendelian inheritance. To date, only one gene has been recognized as a major risk factor for the sporadic form, the APOE gene on chromosome 19, the α4 allele of this gene strongly increasing the risk of developing the disease (Saunders et al, 1993). Furthermore, expression of this gene may be an important determinant for the disease, mutations in the APOE promoter modulating the disease risk (Bullido et al, 1998; Lambert et al, 1998).

More particularly, WO 99/01 574 discloses a method of predicting an increased risk of developing Alzheimer's disease consisting of identifying one or more mutations in the region of genomic ADN regulating the expression of APOE, the mutation(s) being responsible for a modified expression of APOE with respect to a standard population or a modified expression of one allele of APOE with respect to the other.

The mutation is namely a G→T replacement in the promoter region of the APOE gene located 186 bases upstream of the TATA box of said gene, in a potential binding site of the transcription factors Th1/E47CS.

However, APOE locus can only explain less than 50 % of the Alzheimer's disease cases (Farrer et al, 1997) and other genetic determinants should exist.

Recently, three reports described a novel locus on chromosome 12 (Pericak-Vance et al, 1997 ; Wu et al, 1998 ; Rogaeva et al, 1998). Two very plausible candidates genes have been studied in the region of interest: the α2-macroglobulin and the lipoprotein -receptor-related protein (LRP) (Blacker et al, 1998 ; Lendon et al, 1997). However, numerous data seem to exclude the first in sporadic cases, while the second could be only a minor contributor to the disease risk (Lambert et al, 1998 ; Wavrant-de Vrièze et al, 1999). So, the gene located on chromosome 12 and implicated in Alzheimer's disease, is still unknown.

According to the present invention, it has now been discovered that the gene located on chromosome 12 and implicated in Alzheimer's disease is the CP2/LSF/LBP-1 gene previously identified and characterized (Lim et al, 1992 ; Swendeman et al, 1994).

The CP2/LSF/LBP-1 gene comprises 15 exons and encodes a cellular transcription factor that interacts with the α-globulin promoter (Lim et al, 1992) as well as with additional cellular and viral promoter elements including the γ-fibrinogen promoter (Chodosh et al, 1988), the promoter of HIV-1 (Kato et al, 1991) and the SV-40 major late promoter (Huang et al, 1990).

Recently it has been reported to enhance the transcription of the serum amyloid A3 (SAA3) gene which encodes a major acute-phase protein synthesized and secreted manly by the liver (Bing et al, 1999).

It has further been reported that the CP2/LSF/LBP-1 factor interacted with the Fe65 protein, suspected to control the intemalisation and the processing of the APP (Lambrano et al, 1998).

However, its implication with Alzheimer's disease at a genetic level has not been suggested prior to the instant invention.

The inventor's studies that have led to the instant invention show that mutations in the DNA sequence of the CP2/LSF/LBP-1 gene including the region controlling the expression of said gene, namely the prompter substantially increase the risk of a human subject of developing Alzheimer's disease.

The inventors have namely identified numerous silent polymorphisms, particularly polymorphisms located in untranslated regions of the CP2/LSF/LBP-1 gene.

The polymorphisms were namely located in the promoter, the 5' untranslated region of exon 1 in the 3' untranslated region of exon 15.

One of the polymorphisms located in exon 15 of the untranslated region revealed to be correlated with a substantial increased risk of developing Alzheimer's disease.

This polymorphism consists in a G→A mutation at position +15 of the stop codon of the CP2/LSF-LBP-1 gene.

It is herein described a method of predicting an increased risk of a human subject of developing Alzheimer's disease comprising assaying for a mutation within the DNA sequence of the CP2/LSF/LBP-1 gene including the region controlling the expression of said gene.

A mutation may consist of a simple base replacement, an insertion or deletion of one or more bases.

In particular, a mutation includes an allelic variant or polymorphic form of the CP2/LSF/LBP-1 gene locus, defined with reference to the most frequent allele in the general population.

When present in the coding sequence, the mutation may or may not result in an amino acid substitution, deletion or addition.

The mutation may be present in an non coding sequence of the gene. Thus, the mutation may be present in the regulatory sequences of the gene, namely in the promoter of the gene.

The mutation according to the invention is located in the 3' untranslated region of exon 15 of the CP2/LSF/LBP-1 gene.

Accordingly, the method of the invention consists of identifying the presence of a base other than G at position +15 of the stop codon of exon 15 of the CP2/LSF/LBP-1 gene, namely the presence of an Adenine residue in said position.

In order to detect the presence of a CP2/LSF/LBP-1 allele predisposing to Alzheimer's disease, a biological sample such as blood is prepared and analyzed for the presence or absence of susceptibility alleles of CP2/LSF/LBP-1 gene.

Initially the screening method involves amplification of the relevant CP2/LSF/LBP-1 sequences.

In a preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

In one preferred embodiment, the method of the invention comprises the amplification of the region of interest suspected to comprise said mutation, by a polymerase chain reaction by means of oligonucleotide primers, wherein the reaction product comprises polynucleotides of at least 25 contigous nucleotides from said CP2/LSF-LBP-1 gene.

The method may further comprise sequencing gel reaction products from a PCR or RT-PCR reaction, namely the PCR *Bsp*12861digest from the PCR or RT-PCR reaction.

When the probes are used to detect the presence of the target sequences (for example, in screening for Alzheimer's disease susceptibility), the biological sample to be analyzed, such as blood, may be treated, if desired, to extract the nucleic acids. The sample nucleic acid may be prepared in various ways to facilitate detection of the target sequence ; e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 12. Therefore, high stringency conditions are generally desirable in order to prevent false positives.

The stringency of hybridization is determined by a number of factors during hybridization and during the washing procedure, including temperature, ionic strength, base composition, probe length, and concentration of formamide. These factors are outlined in, for example, Maniatis et al, 1982 and Sambrook et al, 1989. Under certain circumstances, the formation of higher order hybrids, such as triplexes, quadraplexes, etc may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels and methods for labeling probes and ligands are known in the art, and include, for example, radioactive labels which may be incorporated by known methods (e.g. nick translation, random priming or kinasing), biotin, fluorescent groups, chemiluminescent groups (e.g. dioxetanes, particularly triggered dioxetanes), enzymes, antibodies and the like. Variations of this basic scheme are known in the art, and include those variations that facilitate separation of the hybrids to be detected for extraneous materials and/or that amplify the signal from the labeled moiety. A number of these variations are reviewed in e.g. Matthews & Kricka, 1988 ; Landegren et al, 1988 ; Mittlin, 1989 ; US 4,868,105 and in EP 225,807.

As noted above, non-PCR based screening assays are also contemplated in this invention. One procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a 103-106 increase in sensitivity. For an example relating to the preparation of oligodeoxynucleotide-alkaline phosphatase conjugates and their use as hybridization probes (see Jablonski et al, 1986).

Two-step label amplification methodologies are known in the art. These assays work on the principle that a small ligand (such as digoxigenin, biotin, or the like) is attached to a nucleic acid probe capable of specifically binding CP2/LSF-LBP-1. Allele specific probes are also contemplated within the scope of this example and exemplary allele specific probes include probes encompassing the predisposing mutation.

In one example, the small ligand attached to the nucleic acid probe is specifically recognized by an antibody-enzyme conjugate.

In one embodiment of this example digoxigenin is attached to the nucleic acid probe. Hybridization is detected by an antibody-alkaline phosphatase conjugate which turns over a chemiluminescent substrate. For methods for labeling nucleic acid probes according to this embodiment (see Martin et al, 1990).

In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays (see Rigby et al, 1977 and Nguyen et al, 1992).

It is also contemplated within the scope of this invention that the nucleic acid probe assays of this invention will employ a cocktail of nucleic acid probes capable of detecting CP2/LSF-LBP-1 gene. Thus, in one example to detect the presence of CP2/LSF-LBP-1 gene in a cell sample, more than one probe complementary to CP2/LSF-LBP-1 is employed and in particular the number of different probes in alternatively 2, 3 or 5 different nucleic acid probe sequences. In another example, to detect the presence of mutations in the CP2/LSF-LBP-1 gene sequence in a patient more than one probe complementary to CP2/LSF-LBP-1 is employed where the cocktail includes probes capable of binding to the allele-specific mutations identified in populations of patients with alteration in CP2/LSF-LBP-1. In this embodiment, any number of probes can be used, and will preferably include probes corresponding to the major gene mutations identified as predisposing an individual to Alzheimer's disease. Some candidate probes include probes that include allele-specific mutations.

The determination of the alleles of the newly-identified polymorphisms may alternatively be performed by creating in one of the primers used for the PCR method one cleavage site for a restriction enzyme which does not exist in the ADN of subjects bearing one of the alleles.

The method of the instant invention may also comprise one or more of the following steps:
- determining the genotype of the apolipoprotein E, the presence of at least one copy of the ε4 allele being indicative of an increased risk of developing Alzheimer's disease ;
- determining the genotype of the Th1/E47cs polymorphism, the presence of at least one copy of the G allele (T→G mutation in position -186 from the TATA box of the APOE gene) being indicative of an increased risk of developing Alzheimer's disease.

The genotype of the apolipoprotein E may be determined as disclosed in WO 95/29 257.

The genotype of the Th1/E47cs polymorphism may be determined as disclosed in WO 99/01 574.

A method of screening for an agent capable of treating Alzheimer's disease is also described. Said method comprises contacting a cultured cell line comprising a mutation or an allelic variant of the CP2/LSF-LBP-1 gene with an agent capable of treating Alzheimer's disease and monitoring the expression or processing of proteins encoded by the mutated form or allelic variant of the CP2/LSF-LBP-1 gene. According to the invention, the cultured cell line comprises a G→A polymorphism in position +15 of the stop codon of exon 15 of the CP2/LSF-LBP-1 gene.

The cell line comprising a mutation or allelic variant of the CP2/LSF-LBP-1 gene advantageously consists of a transgenic host.

The transgenic host may be an immortalized eucaryotic cell line such as Neuroblastoma cell lines Kelly.

Alternatively, the transgenic host may be a bacterium.

Preferably, the host is obtained by integrating by means of known procedures a nucleic acid segment encompassing the mutated region of the CP2/LSF-LBP-1 gene or cDNA comprising a mutated region of the CP2/LSF-LBP-1 gene into the host genome.

The instant application also describes isolated nucleic acids comprising at least 15, preferably at least 20 consecutive nucleotides of the cDNA sequence of the LBP-1 gene having at least one of the following mutations or polymorphisms (the cDNA sequence is available on EMBL under access number M8481 0) :
- exon 1 :
   G → A (+ 547 from position +1 of exon 1, - 146 from ATG, and +217 in EMBL n° M84810) ;
- exon 15 :
   G → A (+ 15 from the stop codon and + 1884 in EMBL n° M84810).

In particular, the invention provides an isolated nucleic acid comprising at least 15, preferably at least 20 consecutive nucleotides of the cDNA sequence of the LBP-1 gene having at least the polymorphism in exon 15: G → A (+ 15 from the stop codon and + 1884 in EMBL n° M84810).

An isolated nucleic acid which comprises at least 15, preferably at least 20 consecutive nucleotides having the G (+ 2089) →A polymorphism is also described.

The application further describes isolated nucleic acids comprising:
- at least 15, preferably at least 20 consecutive nucleotides of the promoter sequence of the LBP-1 gene comprising the T (+520) → G polymorphism (previously read G → A). The sequence is available on EMBL under access number U01965.
- at least 15, preferably at least 20 consecutive nucleotides of sequence SEQ ID n° 1 (intron 2) comprising one or more polymorphisms selected from A (+ 175) → G and G (+ 660) → C ;
- at least 15, preferably at least 20 consecutive nucleotides of sequence SEQ ID n° 2 (intron 3) comprising one or more polymorphisms selected from T (+ 210) → C and T (+ 431) → G ;
- at least 15, preferably at least 20 consecutive nucleotides of sequence SEQ ID n° 3 (intron 7) comprising the C (+134) → A polymorphism ;
- at least 15, preferably at least 20 consecutive nucleotides of sequence SEQ ID n° 4 (intron 9) comprising the A (-27) → G polymorphism ;
- at least 15, preferably at least 20 consecutive nucleotides of sequence SEQ ID n° 5 (intron 10) comprising the T (- 14) → C polymorphism.

The nucleotide sequences SEQ ID n° 1-5 are represented in the enclosed sequence listing :

The nucleotides which are the subject matter of the polymorphisms mentioned above are numbered from the nucleotides in bold letters in SEQ ID n° 1 to 5, up to the right when the nucleotide is specified (+) and down to the left when the nucleotide is specified (-).

The almost complete sequence of CP2/LSF-LBP-1 gene is also shown in the enclosed sequence listing, in the form of SEQ ID n° 48 to 58, wherein:
- SEQ ID n° 48 includes the TATA box according to Swendeman et al, 1994 (see the underlined nucleotides), whereas exon 1 begins at nucleotide 805 (in bold in SEQ ID n° 48) ;
- in SEQ ID n° 49, exon 2 begins at nucleotide 6, and intron 2 at nucleotide 158, whereas exon 3 starts at nucleotide 1035, and intron 3 at nucleotide 1112 ;
- in SEQ ID n° 50, exon 4 begins at nucleotide 6, and intron 4 at nucleotide 112 ;
- in SEQ ID n° 51, exon 5 begins at nucleotide 212, and intron 5 at nucleotide 319 ;
- in SEQ ID n° 52, exon 6 begins at nucleotide 459, and intron 6 at nucleotide 612 ;
- in SEQ ID n° 53, exon 7 begins at nucleotide 6, and intron 7 at nucleotide 117, whereas exon 8 begins at nucleotide 742, and intron 8 at nucleotide 831 ;
- in SEQ ID n° 54, exon 9 begins at nucleotide 6, and intron 9 begins at nucleotide 55, whereas exon 10 begins at nucleotide 446 and intron 10 begins at nucleotide 540 ;
- in SEQ ID n° 55, exon 11 begins at nucleotide 871, and intron 11 at nucleotide 962 ; whereas exon 12 begins at nucleotide 3077 ;
- in SEQ ID n° 56, intron 12 begins at nucleotide 1 ;
- in SEQ ID n° 57, exon 13 begins at nucleotide 6, and intron 13 at nucleotide 149 ;
- in SEQ ID n° 58, exon 14 begins at nucleotide 471 and intron 14 at nucleotide 523, whereas exon 15 begins at nucleotide 1235.

All the polymorphisms that have been discovered are listed in table 3 (see Example 1, herebelow). Similarly, it is herein described isolated nucleic acids comprising at least 15, preferably at least 20 consecutive nucleotides of any of introns 1 to 14 as represented in SEQ ID n° 48 to 58, comprising at least one of the polymorphisms described in table 3.

"Isolated nucleic acid" is one which is substantially separated from other cellular components which naturally accompany a native human sequence or protein (e.g, ribosomes, polymerases, many other human genome sequences and proteins). The term embraces a nucleic acid sequence which has been removed from its naturally occurring and includes recombinant or cloned DNA isolates and chemically synthesized analogs or analogs biologically synthesized by heterologous systems.

Polynucleotide compositions include RNA, cDNA, genomic DNA as well as synthetic forms, and mixed polymers and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, intemucleotide modifications such as uncharged linkages (e.g. methyl phosphonates, phosphodiesters, phosphoamidates, carbamates, etc) charged linkages (e.g. phosphorothioates, phosphorodithioates, etc), pendent moieties (e.g. polypeptides), intercalators (e.g. acridine, psoralen, etc), chelators, alkylators, and modified linkages (e.g., alpha anomeric nucleic acids, etc).

It is also described recombinant nucleic acids as hereindefined comprising all or part of the CP2/LSF-LBP-1 region. The recombinant construct may be capable of replicating autonormously in a host cell. Alternatively, the recombinant construct may become integrated into the chromosomal DNA of the host cell. Such a recombinant polynucleotide comprises a polynucleotide of genomic, DNA, semi-synthetic, or synthetic origin which, by virtue of its origin or manipulation, 1) is not associated with all or a portion of a polynucleotide with which it is associated in nature ; 2) is linked to a polynucleotide other than that to which it is linked in nature ; 3) does not occur in nature.

Therefore, recombinant nucleic acids comprising sequences otherwise not naturally occurring are provided by this invention.

According to a further aspect, the invention provides a vector comprising a nucleic acids comprising at least 15, preferably at least 20 consecutive nucleotides of the cDNA sequence of the LBP-1 gene having at least the polymorphism in exon 15 : G → A (+ 15 from the stop codon, as specified hereabove.

The invention further provides host cells transformed with such a vector.

Large amounts of the polynucleotides of the present invention may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually, the polynucleotide constructs will be suitable for replication in an unicellular host, such as yeast or bacteria, but may also be intended for introduction to (with and without integration within the genome) cultured mammalian or plant or other eukaryotic cell lines. The purification of nucleic acids produced by the methods of the present invention is described, e.g. in Sambrook et al, 1989 or Ausubel et al, 1992.

The polynucleotides of the present invention may also be produced by chemical synthesis, e.g. by the phosphoramidite method described by Beaucage & Carruthers, 1981 or the triester method according to Matteuci and Carruthers, 1981 and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native CP2/LSF-LBP-1 protein or from other receptors or from secreted polypeptides of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook et al, 1989 or Ausubel et al, 1992.

An appropriate promoter and other necessary vector sequences will be selected so as to be functional in the host, and may include, when appropriate, those naturally associated with CP2/LSF-LBP-1 genes. Examples of workable combinations of cell lines and expression vectors are described in Sambrok et al, 1989 or Ausubel et al, 1992 ; see also, e.g. Metzger et al, 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization an others. Vectors and promoters suitable for use in yeast expression are further described in Hitzeman et al, EP 73 675A. Appropriate non-native mammalian promoters might include the early and late promoters from SV40 (Fiefs et al, 1978) or promoters derived from murine Moloney leukemia virus, mouse tumor virus, avian sarcoma viruses, adenovirus II, bovine papilloma virus or polyoma. In addition, the construct may be joined to an amplifiable gene (e.g. DHFR) so that multiple copies of the gene may be made. For appropriate enhancer and other expression control sequences, see also *Enhancers and Eukaryotic Gene Expression,* Cold Spring Harbor Press, Cold Spring Harbor, N. Y. (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encodes proteins that a) confer resistance to antibiotics or other toxic substances, e.g. ampicillin, neomycin, methotrexate, etc ; b) complement auxotrophic deficiencies, or c) supply critical nutrients not available from complex media, e. g. the gene encoding D-alanine racemase for Bacilli. The choice of the proper selectable marker will depend on the host cell, and appropriate markers for different hosts are well known in the art.

The vectors containing the nucleic acids of interest can be transcribed *in vitro*, and the resulting RNA introduced into the host cell by well-known methods, e. g. by injection (Kubo et al, 1988), or the vectors can be introduced directly into host cells by methods well known in the art, which; vary depending on the type of cellular host, including electroporation, transfection employing calcium chloride, rubidium chloride, calcium phosphate, DEAE-dextran, or other substances ; microprojectile bombardment ; lipofection ; infection (where the vector is an infectious agent, such as a retroviral genome) ; and other methods (generally, Sambrook et al, 1989 and Ausubel et al, 1992). The introduction of the polynucleotides into the host cell by any method known in the art, including, inter alia, those described above, will be referred to herein as "transformation". The cells into which have been introduced nucleic acids described above are meant to also include the progeny of such cells.

The invention also provides a transgenic non human animal, preferably mammal with germlines or somatic cells comprising a heterologous gene encompassing a mutated CP2/LSF-LBP-1 gene, namely the CP2/LSF-LBP-1 exon 15 mutant as defined above.

The host as well as the transgenic non human animals may be useful for screening for drugs capable of reducing or treating symptoms associated with the Alzheimer's disease in patients in need of such treatment.

The following examples and figures are intended to illustrate the invention.

In the annexed figures,
- Figure 1 represents a comparison between sequences of the APOE promoter region encompassing the -216 GT polymorphism and an HIV regulatory element, containing a CP2/LSF-LBP-1 binding domains (Parado et al, 1995) ; similar consensus sequences were indicated in bold.
- Figure 2 (a) illustrates protein binding of cytoplasmic and nuclear extracts from different cell lines (N : neuroblastoma ; A : astrocytoma ; I : lymphocyte ; H : hepatoma). (b) amplification of CP2 and β-actin mRNA by RT-PCR in the different cell lines.
- Figure 3 (a) illustrates competition of nuclear protein binding by excess of cold oligonucleotides at equilibrium. (b) relative binding of each allele of the CP2 exon 15 polymorphism for an increase of cold oligonucleotides at the equilibrium.
- Figure 4 illustrates cytoplasmic or nuclear protein binding from lymphocytes treated without (-) or with (+) PHA.
- Figure 5(a) illustrates the mRNA expression pattern of the mRNA of the GG and GA phenotypes of exon 15 for lymphocytes of control and Alzheimer's disease cases.
- Figure 5(b) represents the ratio between the amount of the CP2 and β-actin mRNA for lymphocytes of control and Alzheimer's disease cases.

### Example 1 :

### Polymorphisms of CP2/LSF-LBP-1 gene

We sequenced the entire gene of CP2/LSF/LBP-1 using the oligonucleotides as described in Tables 1 and 2.

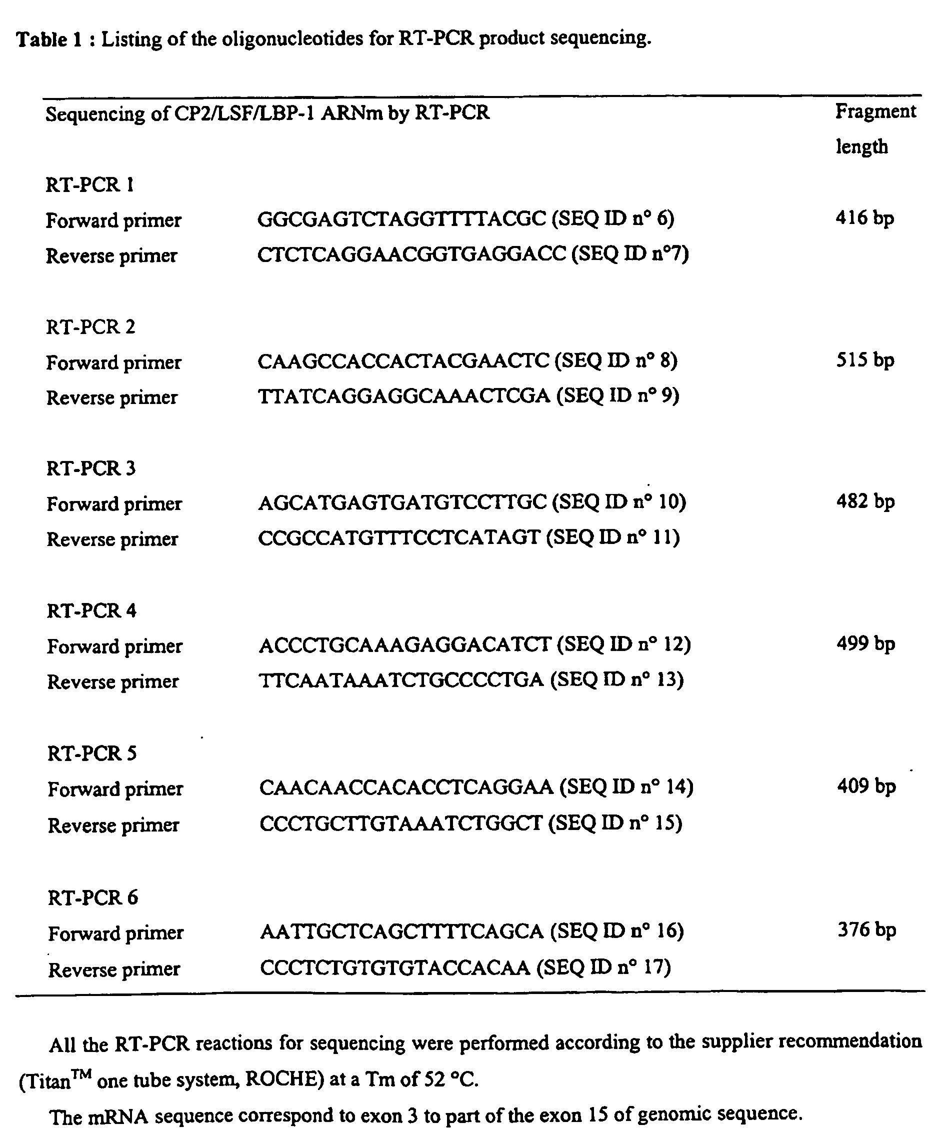

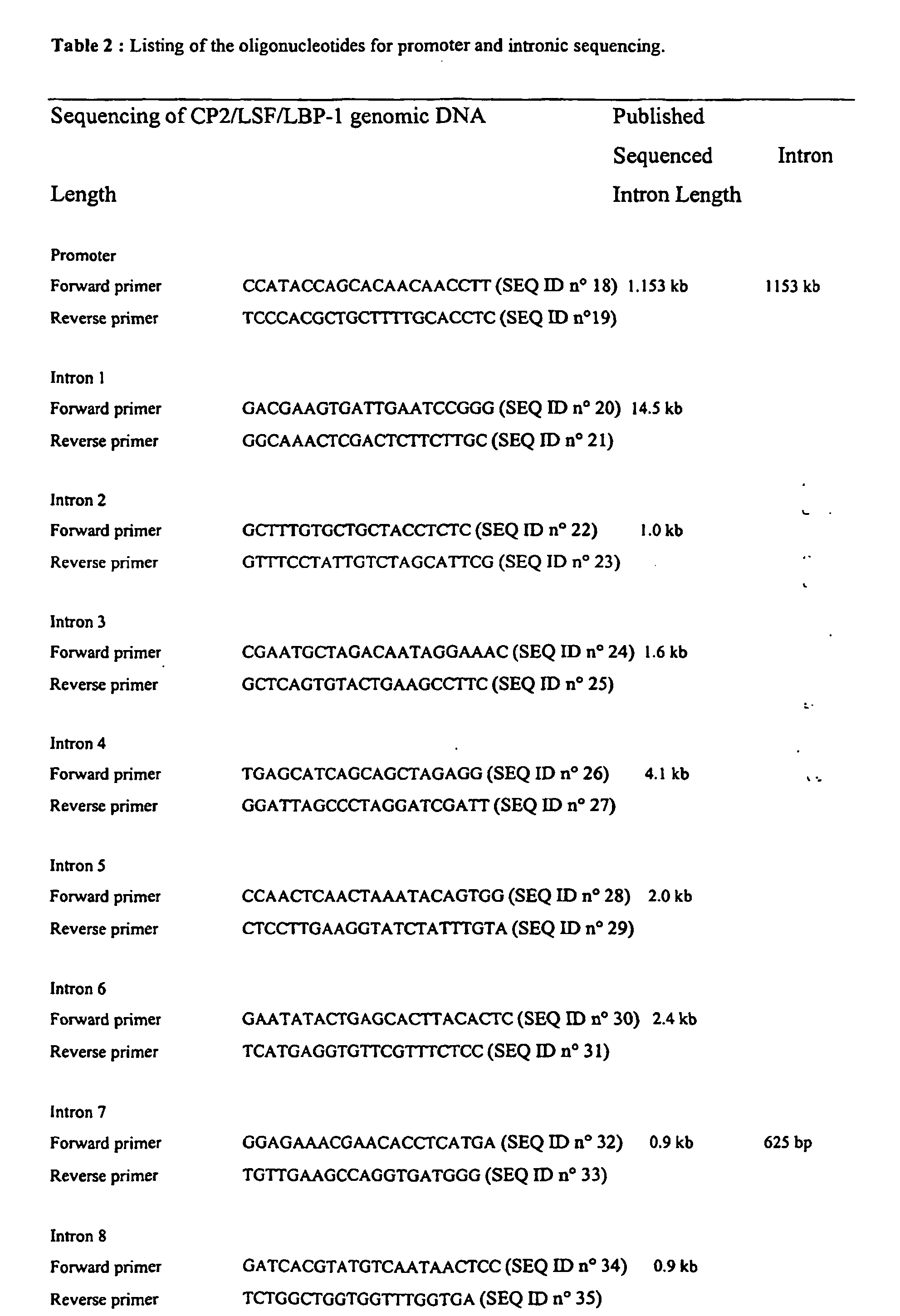

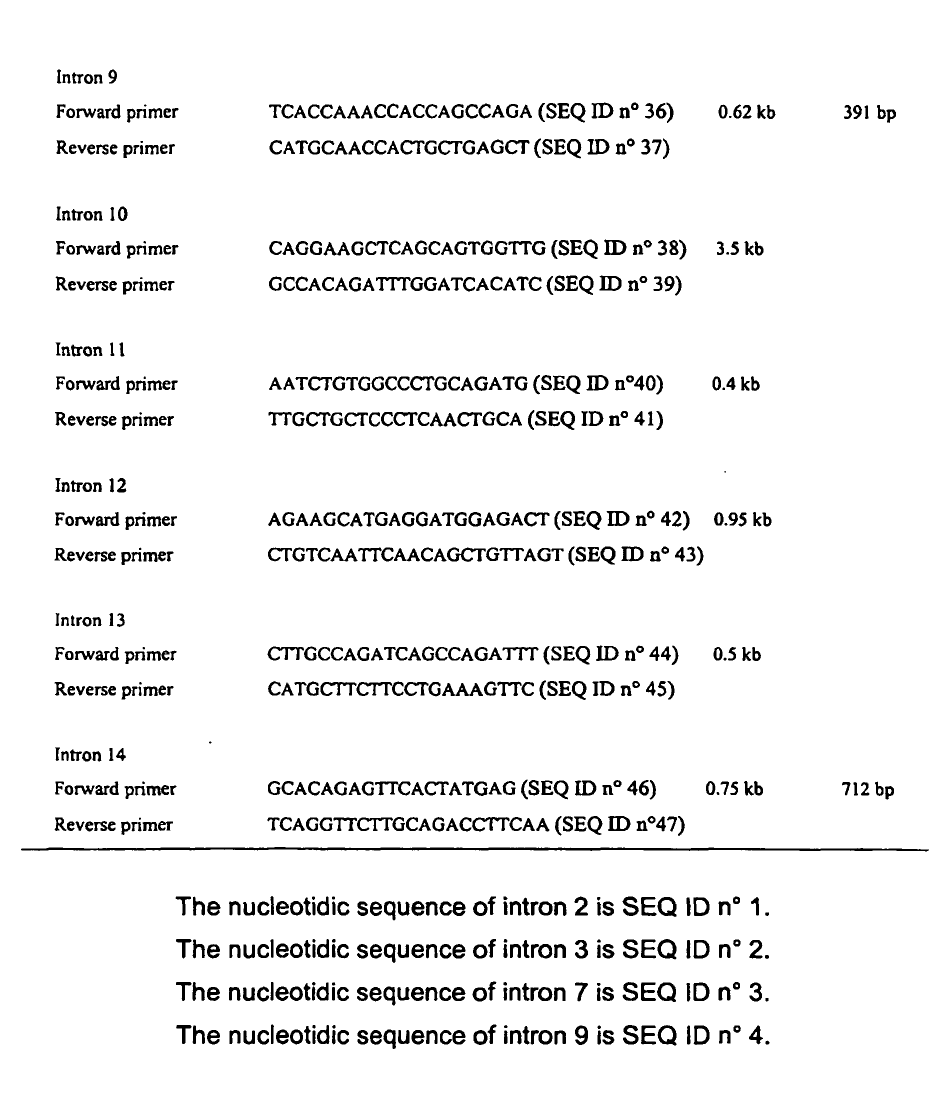

Numerous silent polymorphisms were discovered. The discovered polymorphisms are reported in Table 3.

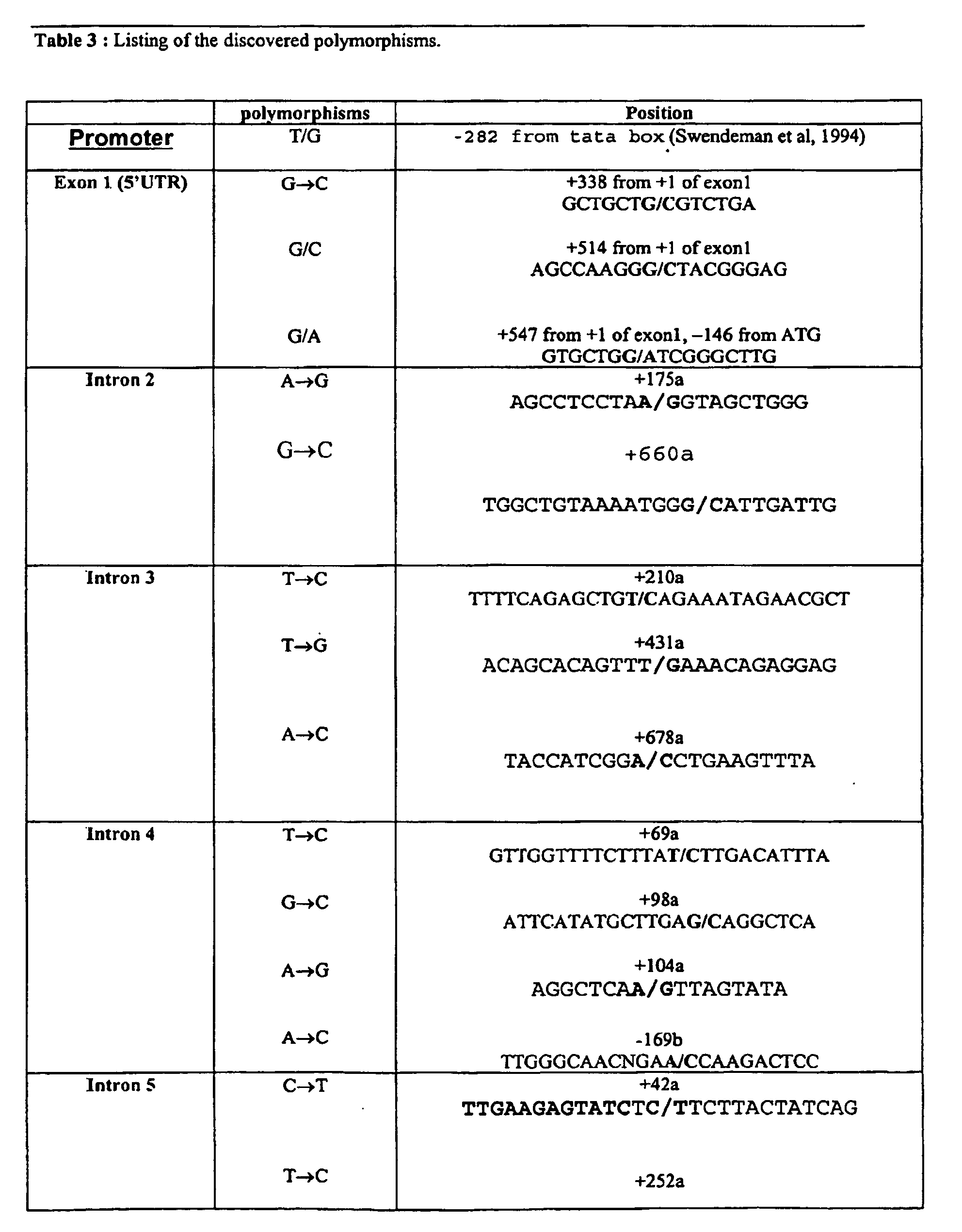

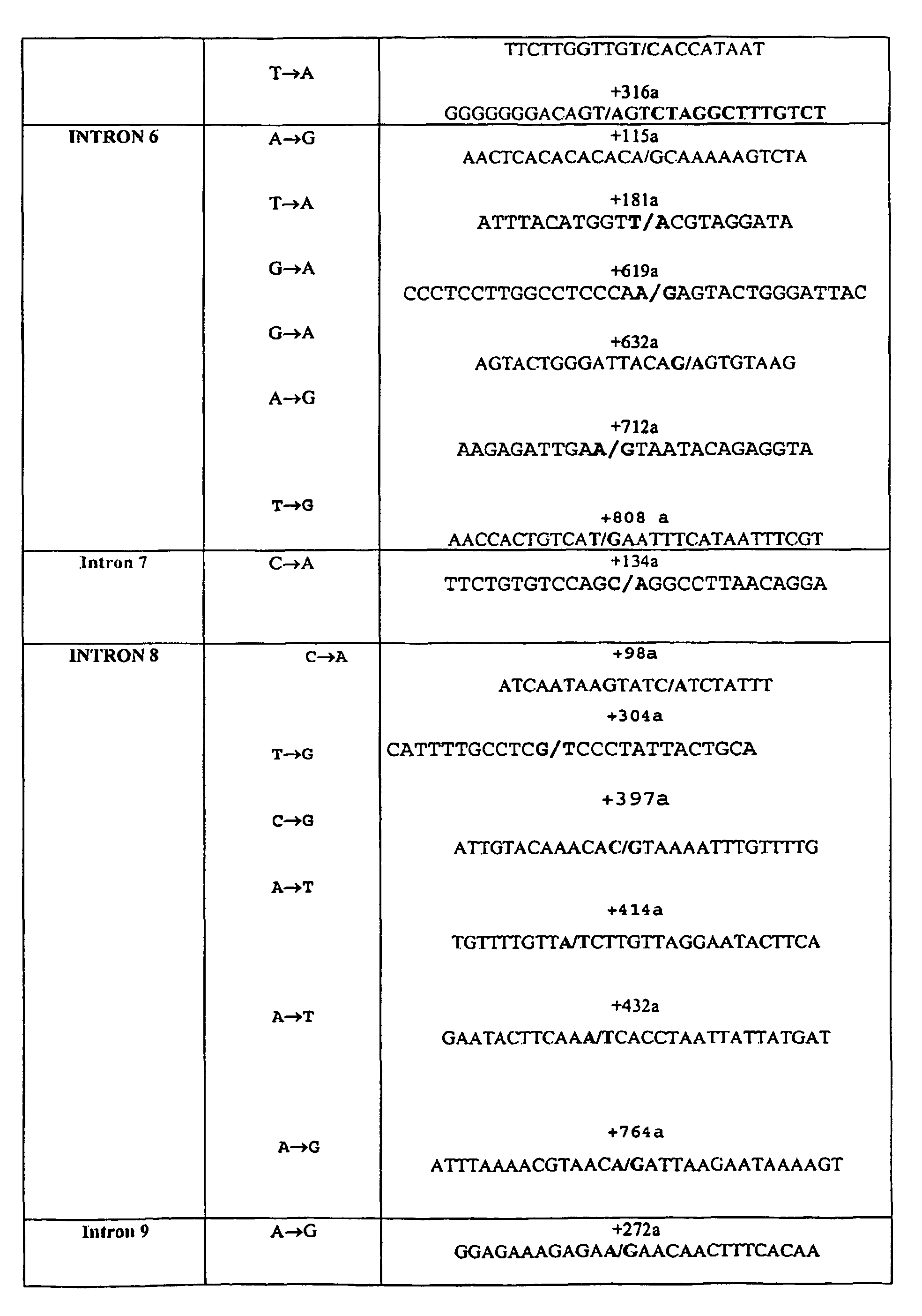

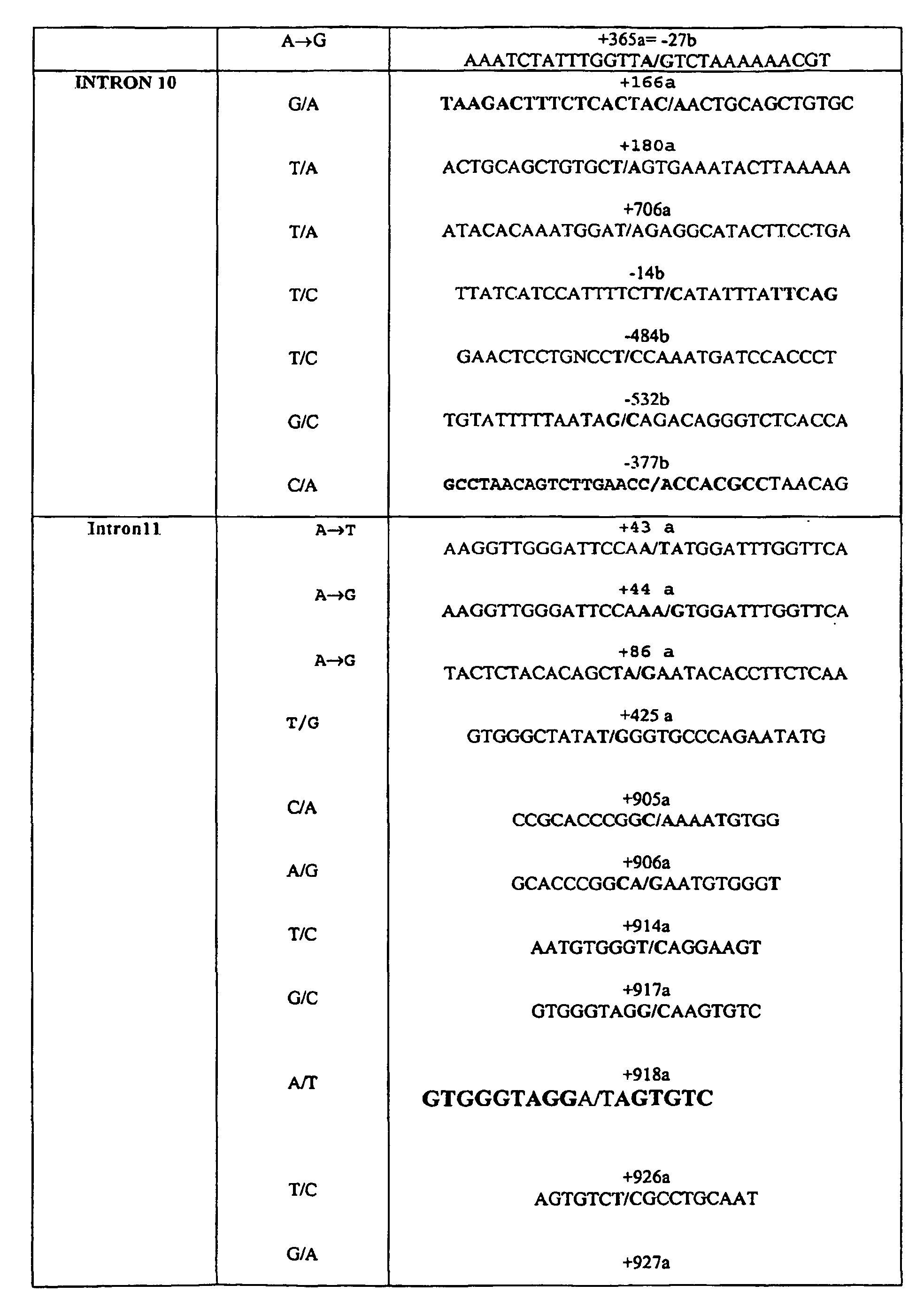

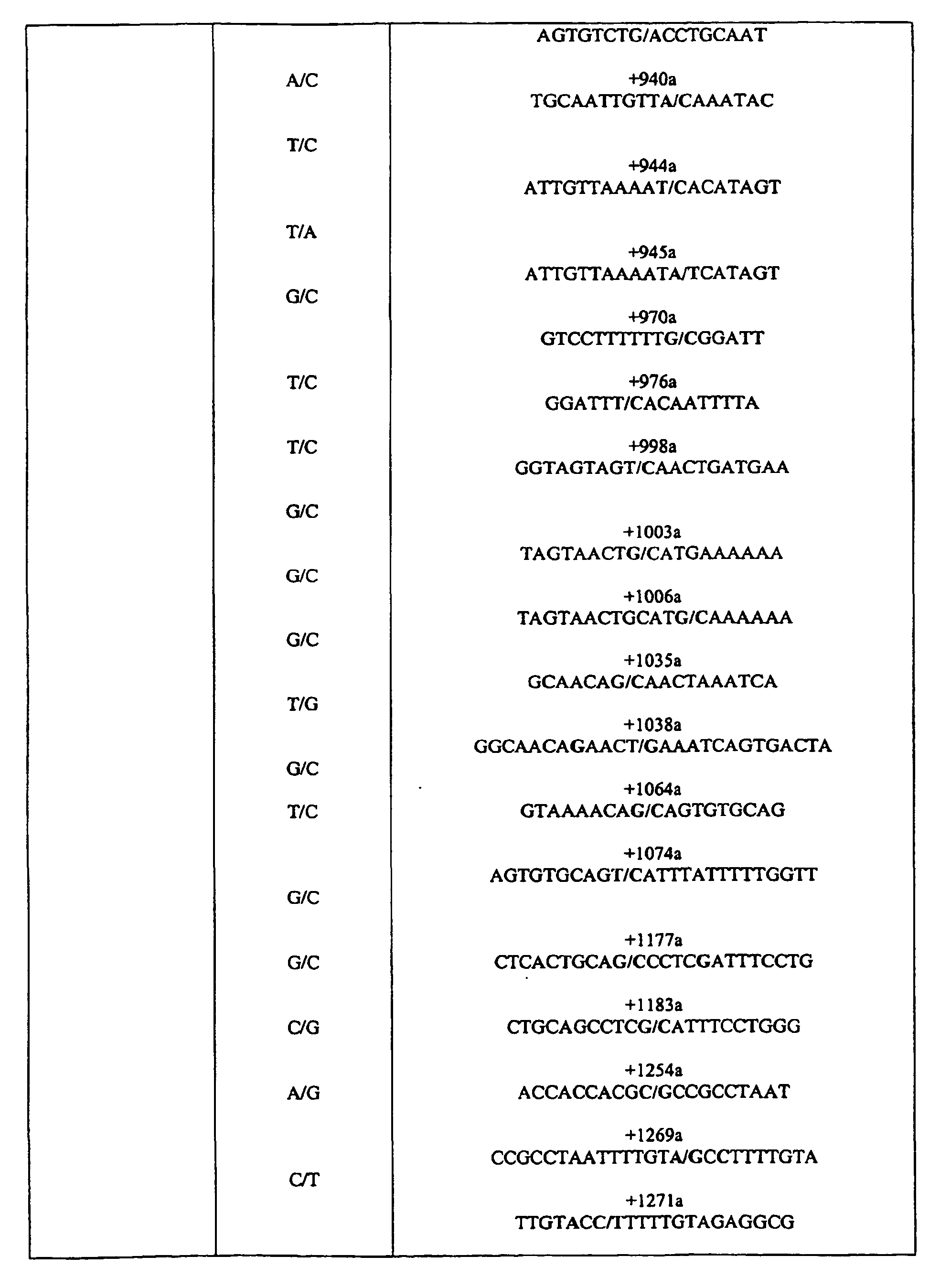

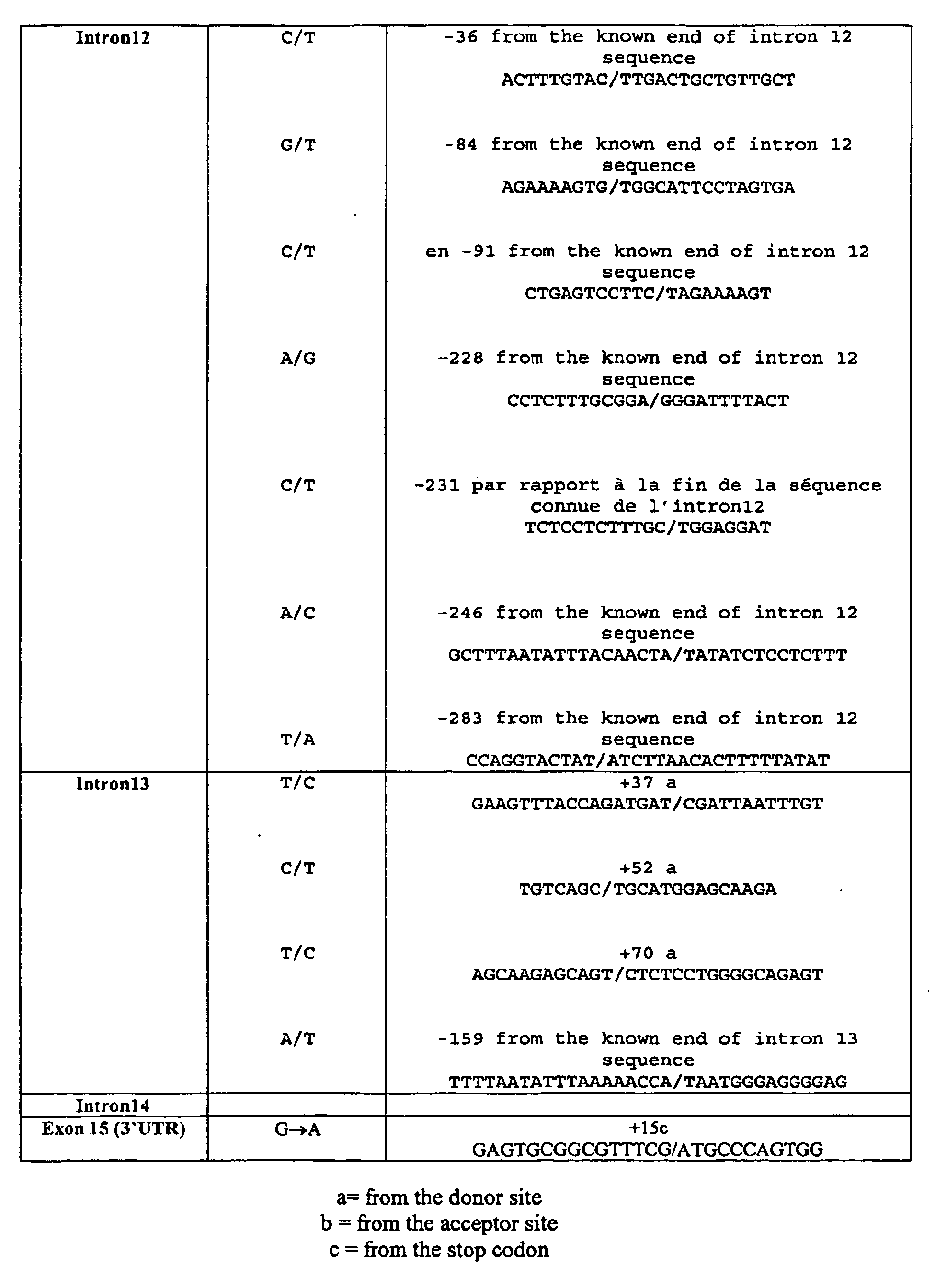

**Table 4 :**

| Frequencies established from Caucasian control population | | | | | | | | | | |
|---|---|---|---|---|---|---|---|---|---|---|
| | | Allele frequency | | | Genotype frequency | | | | | |
| ***APOE*** | n | ε2 | ε3 | ε4 | ε2/ε2 | ε2/ε3 | ε2/ε4 | ε3/ε3 | ε3/ε4 | ε4/ε4 |
| Control cases | 688 | 0.07 | 0.82 | 0.11 | 0.01 | 0.11 | 0.01 | 0.68 | 0.17 | 0.02 |
| AD cases | 709 | 0.04 | 0.62 | 0.34 | 0.00 | 0.03 | 0.04 | 0.40 | 0.41 | 0.12 |

| ***Th1*****/*****E47cs*** | n | G | T | | GG | GT | TT | | | |
|---|---|---|---|---|---|---|---|---|---|---|
| Control cases | 656 | 0.55 | 0.45 | | 0.31 | 0.47 | 0.22 | | | |
| AD cases | 657 | 0.44 | 0.56 | | 0.17 | 0.53 | 0.29 | | | |

| ***CP2pro*** | n | T | G | | TT | TG | GG | | | |
|---|---|---|---|---|---|---|---|---|---|---|
| Control cases | 642 | 0.67 | 0.33 | | 0.44 | 0.45 | 0.11 | | | |
| AD cases | 579 | 0.66 | 0.34 | | 0.44 | 0.44 | 0.12 | | | |

| ***CP2exon1*** | n | G | A | | GG | GA | AA | | | |
|---|---|---|---|---|---|---|---|---|---|---|
| Control cases | 664 | 0.87 | 0.13 | | 0.76 | 0.22 | 0.02 | | | |
| AD cases | 660 | 0.89 | 0.11 | | 0.79 | 0.19 | 0.02 | | | |

| ***CP2exon15*** | n | G | A | | GG | GA | AA | | | |
|---|---|---|---|---|---|---|---|---|---|---|
| Control cases | 680 | 0.94 | 0.06 | | 0.88 | 0.12 | 0.00 | | | |
| AD cases | 704 | 0.97 | 0.03 | | 0.94 | 0.06 | 0.00 | | | |

### Example 2 :

### Implication of CP2 polymorphisms in Alzheimer's disease

We tested in a case-control study three of them located in the promoter (CP2pro), in the exon 1 of the 5'UTR (CP2exon1), in the exon 15 of the 3' untranslated terminal region (CP2exon15).

As already described the presence of the ε4 allele was an important risk factor (OR=5.23 95% CI=4.18-6.77, p<0.0001), while the possession of the ε2 allele exhibited a protective effect (OR=0.49 95% CI=0.34-0.71, *P*<0.0002). The presence of the Th1/E47cs T allele was strongly associated with Alzheimer's disease in this population (OR=2.11 95% CI=1.63-2.73, *P*<0.0001), independently of the ε4 allele. The frequency of the CP2Pro G allele corresponds to 33% of the control group and no variation of allelic and genotypic distributions was observed between patients and controls. The frequency of the CP2Exon1 A allele is of 13% of the control group. No significant variation in the distribution of this polymorphism was observed in cases of Alzheimer's disease compared with controls.

For the CP2exon15 polymorphism, the frequency of the A allele was of 6% in the control group. The frequency of this allele significantly decreased in Alzheimer's disease cases compared with controls (*P*<0.0001). The protective effect for carriers of at least A allele was 0.48 (95% CI=0.33-0.70 ; *P*=0.0002 ; adjusted on age and gender). This effect was stronger in women (OR=0.37 95% CI=0.22-0.63 ; P=0.0002 ; adjusted on age and gender) compared to men (OR=0.64 95% CI=0.36-1.15 ; *P*=0<0.13) (table 4).

### Example 3 :

### Binding analysis of cytoplasmic and nuclear proteins to exon 15 alleles

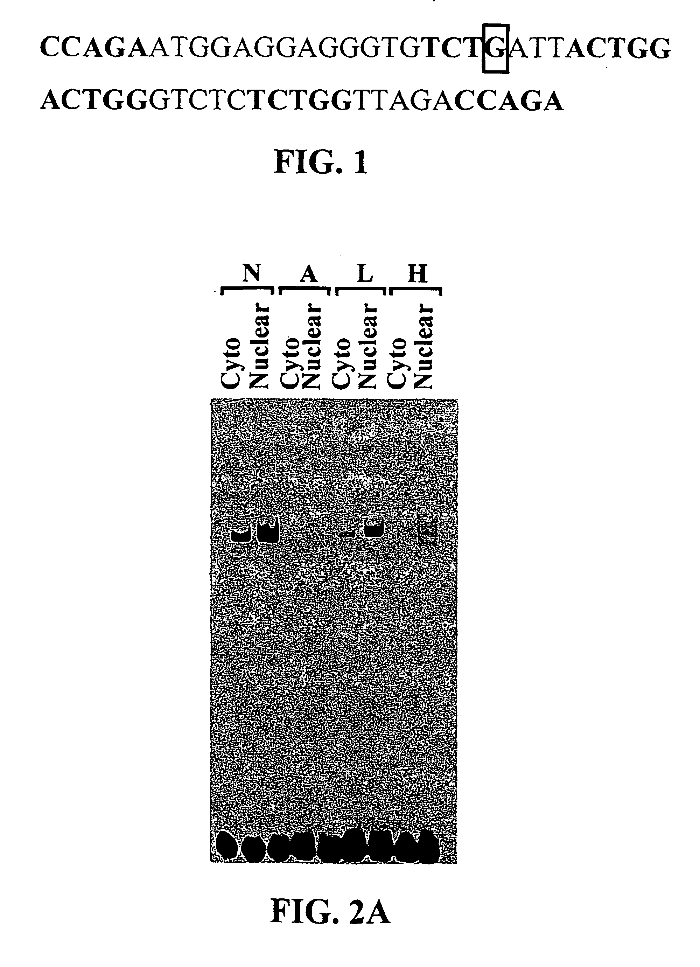

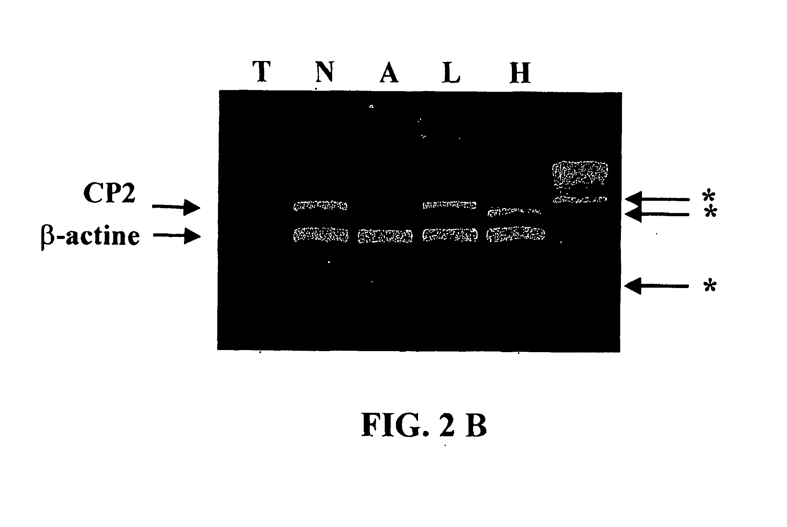

In order to investigate whether the exon15 GA allelic variation may be functional we performed the analyses of binding to cytoplasmic and nuclear proteins by electrophoretic mobility shift assays. We tested different cells lines (fig 2) : neurobastoma (Kelly), astrocytoma (85HG66), hepatoma (HEPG2) cells and lymphocytes from controls and cases of Alzheimer's disease. We observed a protein binding using both cytoplasmic and nuclear extracts from neuroblastoma and lymphocytes. Conversely, the protein binding was weak and not detectable using nuclear extracts from hepatoma and astrocytoma cells, respectively. The CP2 mRNA expression was analysed in these different cell lines and interestingly the presence of CP2 mRNA seems to correlate to the level of binding of the interacting protein (fig. 2b).

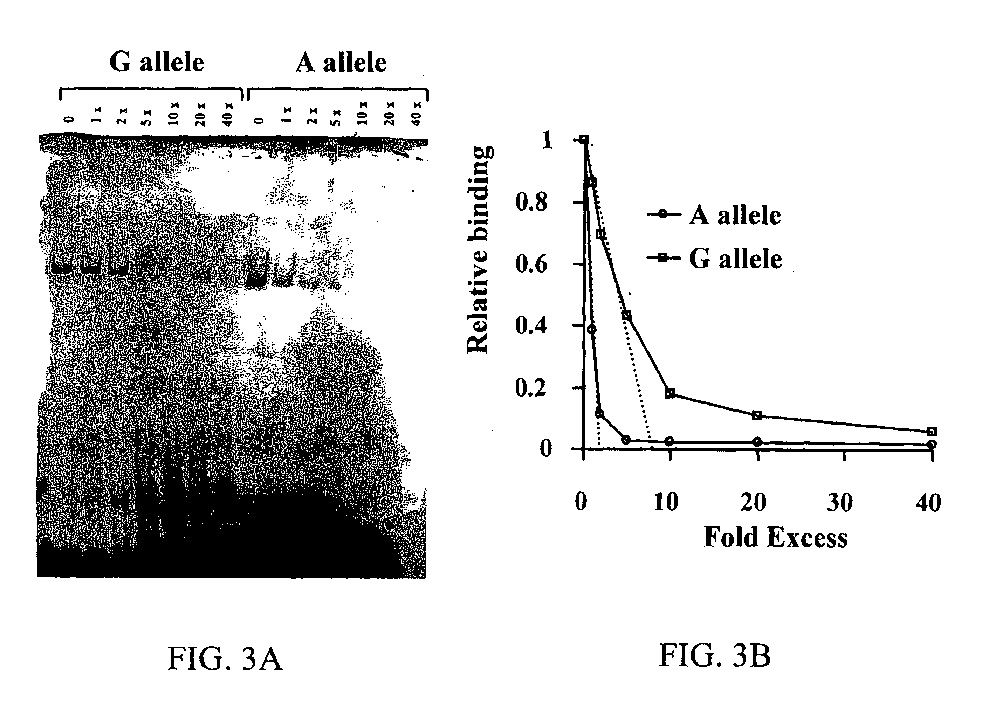

Using nuclear extract from neuroblastoma we tested whether this protein binding could be different according to exon 15 alleles at the equilibrium (Fig 3). We compared the A and G alleles with respect to their affinity for the nuclear proteins. The A allele displayed a 3.75 fold lower affinity (average of 3.5 and 4 fold) to the nuclear proteins than the G allele under equilibrium conditions (fig 3a,b).

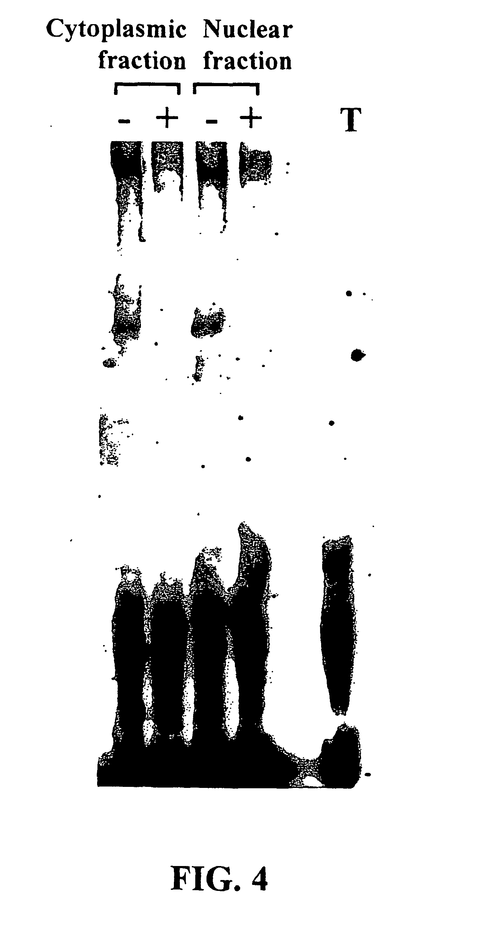

Since the Cp2 is a protein playing a role in the inflammatory process, we tested if the protein binding to the CP2exon15 polymorphic sequence differ after a treatment with Phyto-hemaglutinin (PHA) of lymphocytes from a random population. We observed a high decrease in this binding after such treatment, suggesting that the production of this protein is regulated by the inflammatory process (Figure 4).

### Example 4 :

### Level of expression of CP2 gene in lymphocyte from control and AD patients according to the CP2 exon 15 genotype.

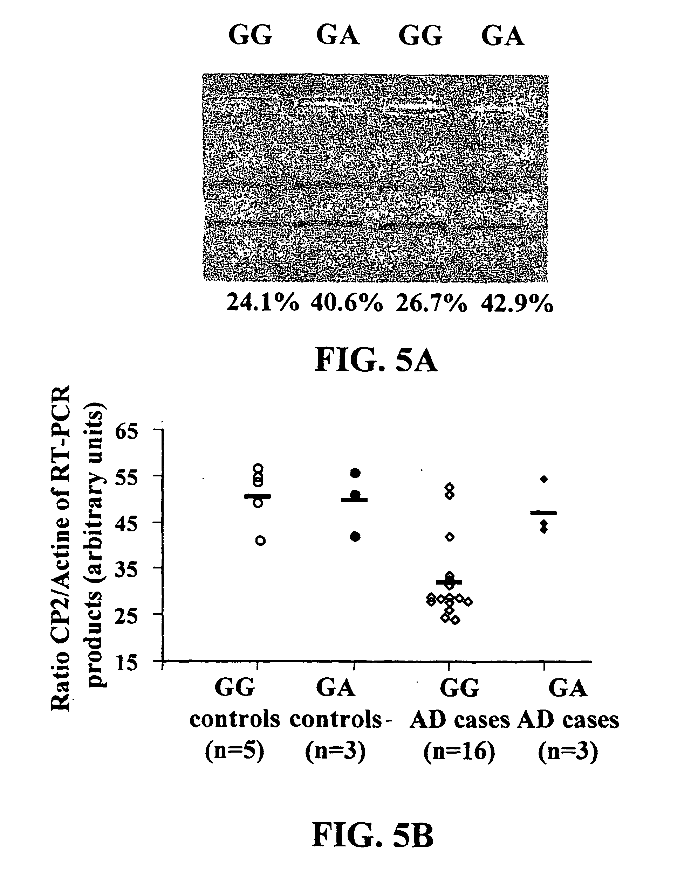

In order to better define the impact of the CP2exon15 polymorphism on the level of expression of the CP2 gene, we estimated by RT-PCR, the ratio between the amount of the CP2 and β-actin mRNA from lymphocytes of control and AD cases (Figure 5). The level of expression of the CP2 gene was decreased in AD cases compared to controls (50.6±6.1 % and 35.9±10.8 % respectively, *P*<0.005 with a non parametric Wilcoxon test). However, AD cases carrying the GA genotype did not exhibit this difference (47.7±6.1 %, *P*<0.9), conversely to those bearing the GG genotype (33.8±10.2 %, *P*<0.002 with a non parametric Wilcoxon test). So, a borderline significant decrease of this expression appeared in the AD cases according to the CP2exon15 genotype (GG=33.8±10.2 versus GA=47.7±6.1, P<0.06 with a non parametric Wilcoxon test).

These data suggested that a decrease of the CP2 expression may be associated with AD development while the protective effect of the A allele may result of a maintenance of the CP2 expression.

### Example 5 :

### Interaction between Th1/E47cs polymorphisms and CP2 polymorphism

Since the Th1/E47cs polymorphism is located in a consensus binding sequence of the CP2 factor, we tested potential interaction between both polymorphisms. We detected a borderline significant interaction (*P*<0.05). Therefore individuals carriers of at least an exon15 A allele and genotyped Th1/E47cs TT presented the weaker risk of developing Alzheimer's disease (OR=0.29 95%CI=0.13-0.64; *P*<0.002), while the carriers of the Th1/E47cs GG genotype did not exhibit a protective effect of the exon15 A allele (OR=0.88 95% CI=0.41-1.11; *P*<0.7), the carriers of the Th1/E47cs GT genotype presenting intermediate impact of the exon 15 A allele (OR=0.48 95% CI=0.28-0.83 ; *P*<0.009). Furthermore, as expected, no increased risk was associated with the Th1/E47cs T allele when individuals carried at least one exon 15 A allele (OR=1.03 95% CI=0.44-2.42; P<0.9), confirming the protective effect of the exon 15 A allele.

### REFERENCES

Bing, Z., Reddy, S.A., Ren, Y., Qin, J. and Liao, W.S. Purification and characterization of the serum amyloid A3 enhancer factor. *J.Biol.Chem.* 274:24649-24656, 1999.

Bullido, M.J., Artiga, M.J., Recuero, M., et al. A polymorphism in the regulatory region of APOE associated with risk for Alzheimer's dementia. *Nat.Genet.* 18:69-71, 1998.

Chodosh, L.A., Baldwin, A.S., Carthew, R.W. and Sharp, P.A. Human CCAAT-binding proteins have heterologous subunits. *Cell* 53:11-24, 1988.

Cruts, M., van Duijn, C.M., Backhovens, H., et al. Estimation of the genetic contribution of presenilin-1 and -2 mutations in a population-based study of presenile Alzheimer disease. *Hum.Mol.Genet*. 7:43-51, 1998.

Farrer, L.A., Cupples, L.A., Haines, J.L., et al. Effects of age, sex, and ethnicity on the association between apolipoprotein E genotype and Alzheimer disease. A meta-analysis. APOE and Alzheimer Disease Meta Analysis Consortium [see comments]. *JAMA* 278:1349-1356, 1997.

Goate, A., Chartier-Harlin, M.C., Mullan, M., et al. Segregation of a missense mutation in the amyloid precursor protein gene with familial Alzheimer's disease [see comments]. *Nature* 349:704-706, 1991.

Huang, H.C., Sundseth, R. and Hansen, U. Transcription factor LSF binds two variant bipartite sites within the SV40 late promoter. *Genes Dev.* 4:287-298, 1990.

Kato, H., Horikoshi, M. and Roeder, R.G. Repression of HIV-1 transcription by a cellular protein. *Science* 251:1416-1479, 1991.

Lambert, J.C., Pasquier, F., Cottel, D., Frigard, B., Amouyel, P. and Chartier-Harlin, M.C. A new polymorphism in the APOE promoter associated with risk of developing Alzheimer's Disease. *Hum.Mol.Genet*. 7:533-540, 1998a.

Lambert, J.C., Wavrant-De, V.F., Amouyel, P. and Chartier-Harlin, M.C. Association at LRP gene locus with sporadic late-onset Alzheimer's disease. Lancet 351:1787-1788, 1998b.

Levy-Lahad, E., Wasco, W., Poorkaj, P., et al. Candidate gene for the chromosome 1 familial Alzheimer's disease locus [see comments]. *Science* 269:973-977, 1995.

Lim, L.C., Swendeman, S.L. and Sheffery, M. Molecular cloning of the alpha-globin transcription factor CP2. *Mol. Cell Biol*. 12:828-835, 1992.

Parada, C.A., Yoon, J.B. and Roeder, R.G. A novel LBP-1-mediated restriction of HIV-1 transcription at the level of elongation in vitro. *J.Biol.Chem*. 270:2274-2283, 1995.

Pericak-Vance, M.A., Bass, M.P., Yamaoka, L.H., et al. Complete genomic screen in late-onset familial Alzheimer disease. Evidence for a new locus on chromosome 12 [see comments]. *JAMA* 278:1237-1241, 1997.

Rogaeva, E., Premkumar, S., Song, Y., et al. Evidence for an Alzheimer disease susceptibility locus on chromosome 12 and for further locus heterogeneity [see comments]. *JAMA* 280:614-618, 1998.

Russo, T., Faraonio, R., Minopoli, G., De Candia, P., De Renzis, S. and Zambrano, N. Fe65 and the protein network centered around the cytosolic domain of the Alzheimer's beta-amyloid precursor protein. *FEBS Lett.* 434:1-7, 1998.

Saunders, A.M., Strittmatter, W.J., Schmechel, D., et al. Association of apolipoprotein E allele epsilon 4 with late-onset familial and sporadic Alzheimer's disease [see comments]. *Neurology* 43:1467-1472, 1993.

Sherrington, R., Rogaev, E.I., Liang, Y., et al. Cloning of a gene bearing missense mutations in early-onset familial Alzheimer's disease [see comments]. *Nature* 375:754-760, 1995.

Swendeman, S.L., Spielholz, C., Jenkins, N.A., Gilbert, D.J., Copeland, N.G. and Sheffery, M. Characterization of the genomic structure, chromosomal location, promoter, and development expression of the alpha-globin transcription factor CP2. *J.Biol.Chem*. 269:11663-11671, 1994.

Wavrant-DeVrieze, F., Lambert, J.C., Stas, L., et al. Association between coding variability in the LRP gene and the risk of late-onset Alzheimer's disease. *Hum.Genet.* 104:432-434, 1999.

Wu, W.S., Holmans, P., Wavrant-DeVrieze, F., et al. Genetic studies on chromosome 12 in late-onset Alzheimer disease [see comments]. *JAMA* 280:619-622, 1998.

## Claims

1. Method of predicting an increased risk of a human subject of developing Alzheimer's disease comprising assaying for a G → A polymorphism in position +15 from the stop codon of exon 15 of the CP2/LFS-LBP-1 gene.

2. A method according to claim 1, comprising the amplification of a region of interest suspected to comprise said mutation, by a polymerase chain reaction by means of oligonucleotide primers, wherein the reaction product comprises polynucleotides of at least 25 contiguous nucleotides from said CP2/LSF-LBP-1 gene.

3. A method according to claim 2, further comprising sequencing gel reaction products from a PCR or RT-PCR reaction, namely the PCR *Bsp* 12861 digest from a PCR or RT-PCR reaction.

4. A method according to anyone of claims 1 to 3 further comprising one or more of the following steps :
- determining the genotype of the apolipoprotein E, the presence of at least one copy of the ε4 allele being indicative of an increased risk of developing Alzheimer's disease ;
- determining the genotype of the Th1/E47cs polymorphism, the presence of at least one copy of the G allele (T→G mutation in position -186 from the TATA box of the APOE gene) being indicative of an increased risk of developing Alzheimer's disease.

5. A method of screening for an agent capable of treating Alzheimer's disease comprising contacting a cultured cell line comprising a G → A polymorphism in position +15 of the stop codon of exon 15 of the CP2/LFS-LBP-1 gene with an agent capable of treating Alzheimer's disease and monitoring the expression or processing of proteins encoded by the polymorph form of the CP2/LSF-LBP-1 gene.

6. An isolated nucleic acid comprising at least 15, preferably at least 20 consecutive nucleotides of the cDNA of the LBP-1, wherein said isolated nucleic acid comprises the position +15 from the stop codon of exon 15 corresponding to position 1285 of SEQ ID n° 58, and wherein the base at said position is an adenine.

7. A vector comprising a nucleic acid according to claim 6.

8. A transgenic host cell comprising a nucleic acid according to claim 7.

9. A transgenic host cell according to claim 8 which is a primary or immortalized eukaryotic cell line.

10. A transgenic host cell according to claim 8 which is a bacterium.

11. A transgenic host cell according to claim 8 wherein the nucleic acid is integrated into the host genome.

12. A transgenic non human animal, preferably mammal with germlines or somatic cells comprising a heterologous gene encompassing a mutated CP2/LSF-LBP-1 gene as defined in claim 1.

## Patentansprüche

1. Verfahren zum Voraussagen eines erhöhten Risikos für einen Menschen, die Alzheimer-Krankheit zu entwickeln, umfassend das Prüfen auf einen G → A Polymorphismus in Position +15 des Stopcodons von Exon 15 des CP2/LFS-LBP-1-Gens.

2. Verfahren nach Anspruch 1, umfassend die Amplifikation einer interessierenden Region, von der vermutet wird, dass sie diese Mutation aufweist, über eine Polymerasekettenreaktion mittels Oligonukleotidprimer, worin das Reaktionsprodukt Polynukleotide von mindestens 25 zusammenhängenden Nukleotiden des CP2/LSF-LBP-1-Gens umfasst.

3. Verfahren nach Anspruch 2, weiterhin umfassend das Sequenzieren von Gelreaktionsprodukten einer PCR oder RT-PCR-Reaktion, nämlich des PCR *Bsp* 12861-Aufschlussprodukts einer PCR oder RT-PCR-Reaktion.

4. Verfahren nach einem der Ansprüche 1 bis 3, weiterhin umfassend einen oder mehrere der folgenden Schritte:
- Bestimmen des Genotyps des Apolipoproteins E, der Gegenwart von mindestens einer Kopie des ε4-Alleles, das ein erhöhtes Risiko zum Entwickeln der Alzheimer Krankheit aufzeigt;
- Bestimmen des Genotyps des Th1/E47cs-Polymorphismus, der Gegenwart von mindestens einer Kopie des G-Alleles (T → G-Mutation in Position -186 von der TATA-Box des APOE-Gens), das ein erhöhtes Risiko zum Entwickeln der Alzheimer Krankheit aufzeigt.

5. Verfahren zum Selektieren eines Mittels, das zur Behandlung der Alzheimer Krankheit geeignet ist, umfassend das Kontaktieren einer kultivierten Zelllinie, umfassend einen G → A Polymorphismus in Position +15 des Stopkodons von Exon 15 des CP2/LFS-LBP-1-Gens, mit einem Mittel, das zur Behandlung der Alzheimer Krankheit geeignet ist, und Beobachten der Expression oder der Entwicklung von Proteinen, die von der polymorphen Form des CP2/LSF-LBP-1-Gens kodiert werden.

6. Isolierte Nukleinsäure, umfassend mindestens 15, vorzugsweise mindestens 20, konsekutive Nukleotide der cDNA des LBP-1, worin die isolierte Nukleinsäure die Position +15 des Stopkodons von Exon 15 umfasst, die der Position 1285 von SEQ ID Nr. 58 entspricht, und worin die Base an dieser Position Adenin ist.

7. Vektor, umfassend eine Nukleinsäure nach Anspruch 6.

8. Transgene Wirtszelle, umfassend eine Nukleinsäure nach Anspruch 7.

9. Transgene Wirtszelle nach Anspruch 8, die eine primäre oder immortalisierte eukaryotische Zelllinie ist.

10. Transgene Wirtszelle nach Anspruch 8, die ein Bakterium ist.

11. Transgene Wirtszelle nach Anspruch 8, worin die Nukleinsäure in das Wirtsgenom integriert ist.

12. Transgenes nicht-menschliches Tier, vorzugsweise Säugetier, mit Keimbahnen oder somatischen Zellen, umfassend ein heterologes Gen, das ein mutiertes CP2/LSF-LBP-1-Gen, wie in Anspruch 1 definiert, enthält.

## Revendications

1. Procédé de prédiction chez un sujet humain d'un risque accru de développer la maladie d'Alzheimer, comprenant la recherche d'un polymorphisme G → A au niveau de la position + 15 à partir du codon stop de l'exon 15 du gène CP2/LSF-LBP-1.

2. Procédé selon la revendication 1, comprenant l'amplification d'une région d'intérêt soupçonnée de comprendre ladite mutation, par une amplification de polymérisation en chaîne (PCR) au moyen d'amorces oligonucléotidiques, dans lequel le produit de la réaction comprend des polynucléotides d'au moins 25 nucléotides contigus dudit gène CP2/LSF-LBP-1.

3. Procédé selon la revendication 2, comprenant, en outre, le séquençage sur gel des produits de réaction issus d'une amplication par polymérisation en chaîne (PCR) ou d'une PCR inverse (RT-PCR), à savoir le produit PCR digéré par *Bsp* 12861, issu d'une réaction PCR ou RT-PCR.

4. Procédé selon l'une quelconque des revendications 1 à 3 comprenant, en outre, l'une ou plusieurs des étapes suivantes consistant à :
- déterminer le génotype de l'apolipoprotéine E, la présence d'au moins une copie de l'allèle ε4 étant indicatrice d'un risque accru de développer la maladie d'Alzheimer ;
- déterminer le génotype du polymorphisme Th1/E47cs, la présence d'au moins une copie de l'allèle G (mutation T → G au niveau de la position -186 à partir de la boîte TATA du gène APOE) étant indicatrice d'un risque accru de développer la maladie d'Alzheimer.

5. Procédé de criblage d'un agent capable de traiter la maladie d'Alzheimer comprenant la mise en contact d'une lignée de cellules cultivées comprenant un polymorphisme G → A au niveau de la position + 15 à partir du codon stop de l'exon 15 du gène CP2/LSF-LBP-1 avec un agent capable de traiter la maladie d'Alzheimer et la surveillance de l'expression ou du traitement des protéines codées par la forme polymorphe du gène CP2/LSF-LBP-1.

6. Acide nucléique isolé comprenant au moins 15, de préférence au moins 20 nucléotides consécutifs de l'ADNc du LBP-1, où ledit acide nucléique isolé comprend la position + 15 à partir du codon stop de l'exon 15 correspondant à la position 1285 de la SEQ ID n° 58, et où la base à ladite position est une adénine.

7. Vecteur comprenant un acide nucléique selon la revendication 6.

8. Cellule hôte transgénique comprenant un acide nucléique selon la revendication 7.

9. Cellule hôte transgénique selon la revendication 8, qui est une lignée de cellules eucaryotes primaires ou immortalisées.

10. Cellule hôte transgénique selon la revendication 8, qui est une bactérie.

11. Cellule hôte transgénique selon la revendication 8, dans laquelle l'acide nucléique est intégré dans le génome de l'hôte.

12. Animal transgénique non humain, de préférence un mammifère ayant des lignées germinales ou des cellules somatiques comprenant un gène hétérologue comprenant un gène CP2/LSF-LBP-1 muté, tel que défini dans la revendication 1.


### SEQUENCE LISTING

<110> Institut Pasteur de Lille INSERM
<120> Implication of a known gene named CP2/LSF-LBP-1 in Alzheimer's disease
<130> BET 99/1159
<140>
   <141>
<160> 58
<170> PatentIn Ver. 2.1
<210> 1
   <211> 910
   <212> DNA
   <213> Homo sapiens
<220>
   <221> intron
   <222> (35)..(910)
<400> 1
   ctccatgatg aaaccctaac gtatctcaat caaggttaga tgcttcctca ttcattagta 60
   ctattgcaga cctactgtat ttatgatttt tttgtttgtt tgtttgtttt gagacggcgt 120
   cacccaggct ggagtgtagt ggcgccatct tggctcactg caagctctgc ctcccgggtt 180
   cacaccattc tcctgcctca gcctcctaag tagctgggac cacaggcacc cgccaccaca 240
   cccggctaat ttttttgtat ttttagtaga gatagggttt catcgtgtta gccaggatgg 300
   tctcgatctc ctgacctcat gatccgcctg ccttggcctc ccaaagtgct gggattacag 360
   gcgtgagcca ccgtgcccgg ccatttatga tgttttatta ggtacactta tatgtaaaaa 420
   cttagatctt tctagtgtgg cctatcaaac tagaagtcct acttcagaga tttgagttct 480
   aatgtcatac ttgatatcta ttactagctt cttttgctta gctgtccatt tttagtgtcc 540
   agcttcctct tcctaatcat attccctaga cacttccatt ggttactggc tgagaagtat 600
   tactctacct ggctctatta tttaccaatt gtgtggcctt ggacaaagtt gctaaaactc 660
   ttttaagttt caatttcttt ggctgtaaaa tgggattggg attgataata ccttagaaaa 720
   gtatatcagt taaatgagat aatgtgacaa tacttagcac agtgctgagc atcttgtaaa 780
   ggcttctgat tgttgttact aatactaata atattattat actgttgtat attcaaccaa 840
   ctgaatttta ttctttccga atctagcaaa gttaatttgt ggtgaatata atatgtaatt 900
   actttttctt                                                         910
<210> 2
   <211> 586
   <212> DNA
   <213> Homo sapiens
<220>
   <221> intron
   <222> (13)..(486)
<400> 2
   aaattggtga aggtaaaacg aataaatcat tcttctgtac aatatttttt actaatcctt 60
   ttgggatttt aaattttgtt gttgttgtta atgttctttt gcttccaatg gcttaatggt 120
   atctcttgcc tgcagtaatt tgttggcaga gtagtaagct gatgtgttat ttctttgggg 180
   tcagagtaag ccccagtatt cacatgaatt tttcagagct gtagaaatag aacgcttacc 240
   actccctgtc cagggtaatc acccctggct cagtgaagtt cttttgggtg gagtaaagta 300
   acctccttga gatctttaga gaagcctcag tgtctttttt gatacttagt cttatagcaa 360
   ggaaaacaga agctactttg atgaagagca gttctcttag tctagattaa cagttaggat 420
   tttgataatt cacagcacag ttgaaacaga ggagttttgt caaaaggtgg tgggaggaat 480
   taataggcac ttctacaaag taagagtaac aaaaggaaag ttgatggaaa caggctctac 540
   ctaccctcan tgtttacagg ttgtatggtg ggttagcaag ccgggg                586
<210> 3
   <211> 713
   <212> DNA
   <213> Homo sapiens
<220>
   <221> intron
   <222> (47)..(671)
<400> 3
   gtggaganat acaggccttc cnatgaagac aaccatactc acagaggtaa aaagatttct 60
   tttggtgaca atttcangtt cataattttt aatcttaaaa attcatcact tccaaactgg 120
   tcagaattta cttctcctaa gccttgaggg acacagtatc acatggattc tgtgtccagc 180
   ggccttaaca ggaagattgc tttagaattt ggcacgaacc atgccactgt ctctgtgtcc 240
   aagttatctt tccccaaatt actctggttt ggtttggttt gctgcaggag ctattgtggt 300
   gttctttgct ttgtatacat aagcacatct cttgcccaaa tagaattcag tttcatcttg 360
   ggcataaacc accttcaatt ttaggaagag ctgtgtgctg cctttggttc tggaacccac 420
   ttataaccaa caaaaatggc ctgggaccgt agttttctaa gacttatttc tttttagaag 480
   tcctattctc aagcaggcct ccccacaggc tccaagatgg cggaaagaga agcagtgttc 540
   tctattgctt atgggagact ctgaggaatt aagatttact aatcaagatg tgctattcaa 600
   gtaacaatgt tttagtcaaa tcctggggtc atatgtgctc gttcattatc taacttatcc 660
   tttgttttta gtgttctcca tggcccgaga tcacgtatgt ccgataactc aaa        713
<210> 4
   <211> 451
   <212> DNA
   <213> Homo sapiens
<220>
   <221> intron
   <222> (19)..(349)
<400> 4
   ncccccctcc gnccagatgt aagtctaaag ttgaatgtca gttacttgca atgtctggta 60
   atactttgat gactgtccaa ttcagtagaa atgcccaact tttagggagt attattcatt 120
   cattcattaa acaaacattt attgaatttt gactatgaac cagatactgt gctagggact 180
   aaagatatga aacaagtaat gcaggacctt tccatttgag cagcttatag tctagtgggg 240
   aagaaaagga tggaactcct tgagcatctt gcatggttgg agaaagagaa aacaactttc 300
   acaaactagg cctgagtatg gggttgtgtg gggtgtttgg ctatgtgtta cctaatgggg 360
   taacagacaa atctatttgg ttatctaaaa aacgtgctct ccctttcaga acctcttacc 420
   aacaaccaca cctcaggaag ctcagcagtg g                                451
<210> 5
   <211> 438
   <212> DNA
   <213> Homo sapiens
<220>
   <221> intron
   <222> (1)..(368)
<400> 5
   tgctcctggc ccaggatttt ttttttaaga gatggggtct tgctatgttg cccacgccta 60
   acagtcttga actcctaaca gtcgattggc ctcaacaatc ctcctacctc agcttctcga 120
   gtagctggga ctacaggcat gttgtgccca gcttagatag tggaaggtta tttcaggaca 180
   gaaagaacaa cttgcattag acatcatcag tatgatgaaa gcatagggtt aagtggggag 240
   gttaattggg gggaattgag acgggagagg agctaggagc caggtcatga agggccttat 300
   atgccatgaa gaaaattatg gattgtaaca ttctggtaat gaggtgtcct tataatacag 360
   cccattttaa tagtcattaa caaagagaat ctttgccttt atcatccatt ttcttatatt 420
   tattccaggg gcagattt                                              438
<210> 6
   <211> 20
   <212> DNA
   <213> Homo sapiens
<400> 6
   ggcgagtcta ggttttacgc 20
<210> 7
   <211> 21
   <212> DNA
   <213> Homo sapiens
<400> 7
   ctctcaggaa cggtgaggac c 21
<210> 8
   <211> 20
   <212> DNA
   <213> Homo sapiens
<400> 8
   caagccacca ctacgaactc 20
<210> 9
   <211> 20
   <212> DNA
   <213> Homo sapiens
<400> 9
   ttatcaggag gcaaactcga 20
<210> 10
   <211> 20
   <212> DNA
   <213> Homo sapiens
<400> 10
   agcatgagtg atgtccttgc 20
<210> 11
   <211> 20
   <212> DNA
   <213> Homo sapiens
<400> 11
   ccgccatgtt tcctcatagt 20
<210> 12
   <211> 20
   <212> DNA
   <213> Homo sapiens
<400> 12
   accctgcaaa gaggacatct 20
<210> 13
   <211> 20
   <212> DNA
   <213> Homo sapiens
<400> 13
   ttcaataaat ctgcccctga 20
<210> 14
   <211> 20
   <212> DNA
   <213> Homo sapiens
<400> 14
   caacaaccac acctcaggaa 20
<210> 15
   <211> 20
   <212> DNA
   <213> Homo sapiens
<400> 15
   ccctgcttgt aaatctggct 20
<210> 16
   <211> 20
   <212> DNA
   <213> Homo sapiens
<400> 16
   aattgctcag cttttcagca 20
<210> 17
   <211> 19
   <212> DNA
   <213> Homo sapiens
<400> 17
   ccctctgtgt gtaccacaa 19
<210> 18
   <211> 21
   <212> DNA
   <213> Homo sapiens
<400> 18
   ccataccagc acaacaacct t 21
<210> 19
   <211> 22
   <212> DNA
   <213> Homo sapiens
<400> 19
   tcccacgctg cttttgcacc tc 22
<210> 20
   <211> 21
   <212> DNA
   <213> Homo sapiens
<400> 20
   gacgaagtga ttgaatccgg g 21
<210> 21
   <211> 21
   <212> DNA
   <213> Homo sapiens
<400> 21
   ggcaaactcg actcttcttg c 21
<210> 22
   <211> 20
   <212> DNA
   <213> Homo sapiens
<400> 22
   gctttgtgct gctacctctc 20
<210> 23
   <211> 22
   <212> DNA
   <213> Homo sapiens
<400> 23
   gtttcctatt gtctagcatt cg 22
<210> 24
   <211> 22
   <212> DNA
   <213> Homo sapiens
<400> 24
   cgaatgctag acaataggaa ac 22
<210> 25
   <211> 21
   <212> DNA
   <213> Homo sapiens
<400> 25
   gctcagtgta ctgaagcctt c 21
<210> 26
   <211> 20
   <212> DNA
   <213> Homo sapiens
<400> 26
   tgagcatcag cagctagagg 20
<210> 27
   <211> 21
   <212> DNA
   <213> Homo sapiens
<400> 27
   ggattagccc taggatcgat t 21
<210> 28
   <211> 22
   <212> DNA
   <213> Homo sapiens
<400> 28
   ccaactcaac taaatacagt gg 22
<210> 29
   <211> 23
   <212> DNA
   <213> Homo sapiens
<400> 29
   ctccttgaag gtatctattt gta 23
<210> 30
   <211> 23
   <212> DNA
   <213> Homo sapiens
<400> 30
   gaatatactg agcacttaca ctc 23
<210> 31
   <211> 21
   <212> DNA
   <213> Homo sapiens
<400> 31
   tcatgaggtg ttcgtttctc c 21
<210> 32
   <211> 21
   <212> DNA
   <213> Homo sapiens
<400> 32
   ggagaaacga acacctcatg a 21
<210> 33
   <211> 20
   <212> DNA
   <213> Homo sapiens
<400> 33
   tgttgaagcc aggtgatggg 20
<210> 34
   <211> 22
   <212> DNA
   <213> Homo sapiens
<400> 34
   gatcacgtat gtcaataact cc 22
<210> 35
   <211> 20
   <212> DNA
   <213> Homo sapiens
<400> 35
   tctggctggt ggtttggtga 20
<210> 36
   <211> 20
   <212> DNA
   <213> Homo sapiens
<400> 36
   tcaccaaacc accagccaga 20
<210> 37
   <211> 20
   <212> DNA
   <213> Homo sapiens
<400> 37
   catgcaacca ctgctgagct 20
<210> 38
   <211> 21
   <212> DNA
   <213> Homo sapiens
<400> 38
   caggaagctc agcagtggtt g 21
<210> 39
   <211> 21
   <212> DNA
   <213> Homo sapiens
<400> 39
   gccacagatt tggatcacat c 21
<210> 40
   <211> 20
   <212> DNA
   <213> Homo sapiens
<400> 40
   aatctgtggc cctgcagatg 20
<210> 41
   <211> 20
   <212> DNA
   <213> Homo sapiens
<400> 41
   ttgctgctcc ctcaactgca 20
<210> 42
   <211> 21
   <212> DNA
   <213> Homo sapiens
<400> 42
   agaagcatga ggatggagac t 21
<210> 43
   <211> 23
   <212> DNA
   <213> Homo sapiens
<400> 43
   ctgtcaattc aacagctgtt agt 23
<210> 44
   <211> 21
   <212> DNA
   <213> Homo sapiens
<400> 44
   cttgccagat cagccagatt t 21
<210> 45
   <211> 21
   <212> DNA
   <213> Homo sapiens
<400> 45
   catgcttctt cctgaaagtt c 21
<210> 46
   <211> 19
   <212> DNA
   <213> Homo sapiens
<400> 46
   gcacagagtt cactatgag 19
<210> 47
   <211> 22
   <212> DNA
   <213> Homo sapiens
<400> 47
   tcaggttctt gcagaccttc aa 22
<210> 48
   <211> 1622
   <212> DNA
   <213> Homo sapiens
<400> 48
   aaaaaccact gttttttttt tttttttttt ttgagacgga gtttcacttt tgttgcccag 60
   gctggagtgc aatggcgcga tctcggctca ctgcaacctc gcctcctggg ttcaagagat 120
   tcctccatgc ctcagacctc cgagtagctg ggattacagg ccgcgccacc acacctagct 180
   aattagtatt ttagtagaga cgggtttaac atgtggtcag gctggtctgg aactccgagc 240
   tcaaggttaa ttccaccgct cgacctccaa gtgctggatt aacggctaac gcggcctggc 300
   ctccaaatgc gtaggattac acgagttgca tgcccgccct aaaaccacat ttttactgat 360
   ctcatgcttt ttctgttcaa atgcatagag agatgccgag ataaatgaat atacaggaag 420
   tgggacttat gcaggtcttg acctccggag aggtgagttt aaggagacag gagtatgggc 480
   agagcaagcg cacacacgta aggagcgttt gggaagccgt caagagtccg ccaggggtgg 540
   agtgggttca taaggctaga agaagggctc cttccatggc tcagggtgac gacttgatgg 600
   gtagcgaata ccagaactct ggtaactaca cggatacacg tttaaacccg gacacttgca 660
   cgccgggtcc tccccgtgat cgcccagccc atctcggggg cagagccgtc gcagccccgc 720
   ctatcatgct gccgagactg acgcggtctc ttctttatag gcgaacgaca gcgtgacgtc 780
   atgttcgccg ggcaggcgag gaaagaggac gccatgattg gttggcgctg ggcggcggga 840
   cggtggaagg gcctggcgag tctaggtttt acgcctgtgc tggactttct ccttccatgt 900
   ttccaggccg tggggggcta cagagggcga gaagtcggct cagcggaaac ctggatttgg 960
   ttctaagccg tggggttgag aaggggtgac cggaagtgat cgtgggactg accggaagcg 1020
   aggcctggag gggaaagaga gagcgagacc tgggagggag ggggcctcca gcagaaaggg 1080
   gcgggggaaa aggtgcaaaa gcagcgtggg agcgccgggc tggcttcctg cggctgctgc 1140
   tggtctgact gggaagcagc aagccaccac tacgaactct caagaggagt gggagtgcgg 1200
   gagtccagag ctgcctctgg gaagtctgca gtagttgagc aaaggggtcc tcacgttcct 1260
   gagagctggg caggggggat tttggaacct ggggcagcca agaacgagca gccaagggta 1320
   cgggagatta gttgtgcaca gagcagtgct ggtcgggctt gggggtggct ggtgggcact 1380
   gcgtgggaaa ccttggtttg tagttttctt ggtttgcgtt actcctgttg ggtagaatta 1440
   ccctccgcgc ctttgtacaa gacacggtgt ctcctggggc aaggaaggag ccaggatggc 1500
   ctgggctctg aagctgcctc tggccgacga agtgattgaa tccgggttgg tgcaggactt 1560
   tgatgctagc ctgtccggga tcggccagga actgggtgct ggtgcctata gcatgaggta 1620
   ag                                                                1622
<210> 49
   <211> 2205
   <212> DNA
   <213> Homo sapiens
<400> 49
   tttagtgatg tccttgcatt gcccattttt aagcaagaag agtcgagttt gcctcctgat 60
   aatgagaata aaatcctgcc ttttcaatat gtgctttgtg ctgctacctc tccagcagtg 120
   aaactccatg atgaaaccct aacgtatctc aatcaaggtt agatgcttcc tcattcatta 180
   gtactattgc agacctactg tatttatgat tttttgtttt gtttgtttgt tttgagacgg 240
   cgtcacccag gctggagtgt agtggcgcca tcttggctca ctgcaagctc tgcctcccgg 300
   gttcacacca ttctcctgcc tcagcctcct aagtagctgg gaccacaggc acccgccacc 360
   acacccggct aatttttttg tatttttagt agagataggg tttcatcgtg ttagccagga 420
   tggtctcgat ctcctgacct catgatccgc ctgccttggc ctcccaaagt gctgggatta 480
   caggcgtgag ccaccgtgcc cggccattta tgatgtttta ttaggtacac ttatatgtaa 540
   aaacttagat ctttctagtg tggcctatca aactagaagt cctacttcag agatttgagt 600
   tctaatgtca tacttgatat ctattactag cttcttttgc ttagctgtcc atttttagtg 660
   tccagcttcc tcttcctaat catattccct agacactccc attggttact gtctgagaag 720
   tattactcta cctggctcta ttatttacca attgtgtggc cttggacaag ttgctaaaac 780
   tcttttaagt ttcaatttct ttggctgtaa aatgggattg attgataata ccttagaaaa 840
   gtatatcagt taaatgagat aatgtgacaa tacttagcac agtgctgagc atcttgtaaa 900
   ggcttctgat tgttgttact aatactaata atattattat actgttgtat attcaaccaa 960
   ctgaatttta ttctttccga atctagcaaa gttaatttgt ggtgaatata atatgtaatt 1020
   actttttctt ctaggacagt cttatgaaat tcgaatgcta gacaatagga aacttggaga 1080
   acttccagaa attaatggca aattggtgaa ggtaaaacga ataaatcatt cttctgtaca 1140
   atatttttta ctaatccttt tgggatttta aattttgttg ttgttgttaa tgttcttttg 1200
   cttccaatgg cttaatggta tctcttgcct gcagtaattt gttggcagag tagtaagctg 1260
   atgtgttatt tctttggggt cagagtaagc cccagtattc acatgaattt ttcagagctg 1320
   tagaaataga acgcttacca ctccctgtcc agggtaatca cccctggctc agtgaagttc 1380
   ttttgggtgg agtaaagtaa cctccttgag atctttagag aagcctcagt gtcttttttg 1440
   atacttagtc ttatagcaag gaaaacagaa gctactttga tgaagagcag ttctcttagt 1500
   ctagattaac agttaggatt ttgataattc acagcacagt ttaaacagag gagttttgtc 1560
   aaaaggtggt gggaggaatt aataggcact tctacaaagg aaagttgatg gaaaacaggc 1620
   tctacctacc ctcagtgttt acaggttgta tggtgggtta gcaagcgggg gagttggtga 1680
   gtttttcata tacatctgaa gagctaagcc agataggccg tggtataaca gaagaggtgt 1740
   gcagaaacgc ttcagacagg agtactttaa tttcctagtt accatcggac tgaagtttaa 1800
   gccaagtttt aaatatggta ccagttaact attcctagtt accatcggac tgaagtttaa 1860
   gcccaagttt taaatatggt accagttaac tataaagatt ctatctgaga aattggctgg 1920
   acccggtggc tcacgcctat gtcccagcac tttgagaggc tgaggcgagg tcgcctgagg 1980
   ttgggagttt gagaccagcc tgaccaagat ggngaaaccc tgtctctaca aaaaatacaa 2040
   aattagtcag gcgtggtagc acatgcctgt aatcccagct actcaggagg ctgaggcagg 2100
   agaattcctt gaacccggga ggtggaggtt gcggtgagcc gagatcacac cattgcactc 2160
   caacctgggc aacaagagca aaatctgttt caaaaaaaaa aaaaa                 2205
<210> 50
   <211> 574
   <212> DNA
   <213> Homo sapiens
<400> 50
   tttagagtat attccgtgtg gtgttccatg acagaaggct tcagtacact gagcatcagc 60
   agctagaggg ctggaggtgg aaccgacctg gagacagaat tcttgacata ggtgagttaa 120
   gagagaatct attgcctagg aaatgccttt agctatggac ccagagttgg ttttctttat 180
   ttgacattta ccttgattca tatgcttgag aggctcaatt agtataaacc ttgaaattat 240
   ttactaagca taaaaaattt taagtaatac aagttaaaat gatatcaaaa ttgaagttgg 300
   aagtgtaaac actttagttc catagcaact ataggttgat tttgttgttt tttaagttgt 360
   ggtaaaatat acattacata aaagttatca ttttaaccat ttttaagtgg atagttcagt 420
   aacattaggt acattcacat tattgtgtaa ccatcactat catccatctc cagaactttt 480
   ctcatctgca aaaccagaag tctgtacctg ttaaacaaca acgccccatt ctcccttctt 540
   cacagcccct ggtaaccact agtctccttt ctgg                             574
<210> 51
   <211> 729
   <212> DNA
   <213> Homo sapiens
<400> 51
   actnggcctn tattacccct gcactccagc ttgggcaacn gaacaagact ccntaaaaaa 60
   caacaacaac gcaaaaaaaa aaacaaagaa atttaggatg tgatatacaa aaatcttggt 120
   agctacattg agtaatataa gaatcaatgc tttcttattt aaaactgagt ggccctcaga 180
   attgttcaat gattaccatt ttgtttctca gatatcccga tgtctgtggg tataatcgat 240
   cctagggcta atccaactca actaaataca gtggagttcc tgtgggaccc tgcaaagagg 300
   acatctgtgt ttattcaggt aaggcgttgc agatagggtt ccagttttga agagtatctc 360
   tcttactatc agtttatttc ttaaaggata aaacatgtta actttgaaat ctttacaggg 420
   ttctagcctg actgtcaagc ccaggctctt taacctgtgc ctgagagcat tgaggagttt 480
   ctgaattgct cttctaatac cccagtttat ttatttgtgt ctcccaaaat tgcttggtaa 540
   tggctgcata atatttcatt tcttggttgt accataattt acataacaat tatttggcag 600
   ttagattggc ataatttttt ttggggggga cagtgtctag gctttgtctc ccaatctgga 660
   gtggagtggt gcaatcttcg ggctcactac aacctctgcc tcccagttca agcgattctc 720
   ttgcctcaa                                                         729
<210> 52
   <211> 1554
   <212> DNA
   <213> Homo sapiens
<400> 52
   aaaaaaaaaa aaaaaaattc accaatctta gactttatgc agtagccaag caaacctttt 60
   tctccactat tgccntgaac tttcatattt actatgttcc cagactgggt agtgactcct 120
   ctttctacaa tcttccttaa aattgaggaa actttaacta acataaatca tttctctttt 180
   gctagctctg tgttctttga agcagttaat gaatttagtt cctcacagta gcttttgtta 240
   gcattagatc tgtactttta ttttgtttgc atttgtaaag catttttcta tttcctctat 300
   cagattgtaa gttcttcaag atcaaaaaga ggaaaagtgt tttttgtagg tattcataat 360
   gcctggtaaa ggttttgctg ttaatagttg ctaaaaaaat actgccaact ggtttggcta 420
   gctagcggta ctgaagcctt gctgttgtat tctttcaggt gcactgtatt agcacagagt 480
   tcactatgag gaaacatggc ggagaaaagg gggtgccatt ccgagtacaa atagatacct 540
   tcaaggagaa tgaaaacggg gaatatactg agcacttaca ctcggccagc tgccagatca 600
   aagttttcaa ggtattcctt gggcaatgtg ctggcccttg gattctttta ccttatcatt 660
   ctatttttaa cattagttac caagtcagaa aatcaacttg tctttttttg aaaactcaca 720
   cacacacaaa aagtctaagc tagagtcatt gaaataatta ccaagatgcc tggtaaagaa 780
   atttacatgg ttcgtaggat aataggaact tggaaaagag ggctagaggc aagaacatgg 840
   attctcattg aaagctctta aagcctattg aagttacaag ctcttgtaaa gctcttgtaa 900
   agctcacaaa aattgactag ttatggttat tttcatactt gatcttttct tatttggtct 960
   tatttggtct tatttattta tttatttatt tttgagactg ggtttcattt tgtcacccag 1020
   gctggagtgc agtgacgcaa tcatagttta acctcctggg ctcaattgat cttccagcgt 1080
   cagcttcccc agtaagtggg actacaggca tgtgccacca caccgggcta atttttgtat 1140
   tttttaatag agagggggtc ttgccatgtt gcccaggctg gccttgaact cccgggctta 1200
   agttatctgc cctccttggc ctcccaaagt actgggatta caggtgtaag ccactgtgcc 1260
   ccagccaggg tcctgtttat ttttatcaca tgaataaata tgttctcatt gtaagagatt 1320
   gaataataca gaggtattca tagcaacact gaaattctcc ctttactact cttacactga 1380
   ccccctccac aacctcttcc cagaggaaac cactgtcata atttcataat ttcgtatgtg 1440
   ttcttctacg tccctcttct tttttttttt tgattcggag tttcagtctg acacccaggc 1500
   cgnanttcaa tggtaaattc ccaaccaaag gnaacccccn tccccggggt taaa       1554
<210> 53
   <211> 1831
   <212> DNA
   <213> Homo sapiens
<400> 53
   ttcagcccaa aggtgcagac agaaagcaaa aaacggatag ggaaaaaatg gagaaacgaa 60
   cacctcatga aaaggagaaa tatcagcctt cctatgagac aaccatactc acagaggtaa 120
   aaagatttct tttggtgaca atttcangtt cataattttt aatcttaaaa attcatcact 180
   tccaaactgg tcagaattta cttctcctaa gccttgaggg acacagtatc acatggattc 240
   tgtgtccagc ggccttaaca ggaagattgc tttagaattt ggcacgaacc atgccactgt 300
   ctctgtgtcc aagttatctt tccccaaatt actctggttt ggtttggttt gctgcaggag 360
   ctattgtggt gttctttgct ttgtatacat aagcacatct cttgcccaaa tagaattcag 420
   tttcatcttg ggcataaacc accttcaatt ttaggaagag ctgtgtgctg cctttggttc 480
   tggaacccac ttataaccaa caaaaatggc ctgggaccgt agttttctaa gacttatttc 540
   tttttagaag tcctattctc aagcaggcct ccccacaggc tccaagatgg cggaaagaga 600
   agcagtgttc tctattgctt atgggagact ctgaggaatt aagatttact aatcaagatg 660
   tgctattcaa gtaacaatgt tttagtcaaa tcctggggtc atatgtgctc gttcattatc 720
   taacttatcc tttgttttta gtgttctcca tggcccgaga tcacgtatgt caataactcc 780
   ccatcacctg gcttcaacag ttcccatagc agtttttctc ttggggaagg gtaagtctgg 840
   gaaataaaat tggcttatat ttgtccaaag atatatattc tttcattacc tgttcataaa 900
   caagatcttt tttaaatcaa taagtatctc tatttggtca ttctttgatg gatttanatn 960
   tcaactgaga tttggagtat cttcaaaaga gatcgaaaga gttttatata gccctttctt 1020
   ctctttgtgt tctcccctca cactcctcct cgtctttttt ccccccacta gaaagtagca 1080
   tttatggtca atttttagtg gttcagggac tgataatcag ggcattttgc ctcgccctat 1140
   tactgcacat cttgtggtta tttcatttcc tgatcatact tgcaccatac agtggtcttt 1200
   gggaactgga aacaattgta caaacactaa aatttgtttt gttacttgtt aggaatactt 1260
   caaacaccta attattatga atctttgata attggggaaa taagtttgaa gatgatggct 1320
   aaaatgaagt tttggaatta tttatgggat acatttaatc ttgccttatt atcttttacc 1380
   ataagagata attgaaagtt ggtacagatg cctactccaa ttttaaaaag tagctgtatt 1440
   atagtccaag catttaacat gtatttcaca tagccatatt gaaacttgaa atagttaggc 1500
   taccaaatgt gcgagtatat tgtttggttg caatgtttgt ggttttggat ttatttaggc 1560
   tctatgtctg acctactttt taaaggattt aattaaggta gcttaaatat ttaaaacgta 1620
   acaattaaga ataaaagtag gtattagttg tagagggaat acatataagt agggccctaa 1680
   gataagttaa ttactaagtt cgaataccaa atttgaattt tgcagctaag gctaaaaaag 1740
   gaaaaagatc tttttttaaa agaaaaaaaa atcttaacat ccacatagat gatacattct 1800
   acttataggt aattccaaat atgatatagg g                               1831
<210> 54
   <211> 1658
   <212> DNA
   <213> Homo sapiens
<400> 54
   tttagaaatg gttcaccaaa ccaccagcca gagccacccc ctccagtcac agatgtaagt 60
   ctaaagttga atgtcagtta cttgcaatgt ctggtaatac tttgatgact gtccaattca 120
   gtagaaatgc ccaactttta gggagtatta ttcattcatt cattaaacaa acatttattg 180
   aattttgact atgaaccaga tactgtgcta gggactaaag atatgaaaca agtaatgcag 240
   gacctttcca tttgagcagc ttatagtcta gtggggaaga aaaggatgga actccttgag 300
   catcttgcat ggttggagaa agagaaaaca actttcacaa actaggcctg agtatggggt 360
   tgtgtggggt gtttggctat gtgttaccta atggggtaac agacaaatct atttggttat 420
   ctaaaaaacg tgctctccct ttcagaacct cttaccaaca accacacctc aggaagctca 480
   gcagtggttg catcgaaatc gtttttctac attcacaagg cttttcacaa acttctcagg 540
   ttaaattttc tttttctttt gttttcccta gcatgaaatt ttcctttgca ttctagggcc 600
   tcctggtatc catgggagag ttgtgtgtgt atatgtgtat gagagatctt actgcatttt 660
   tctgatgaaa tgaatttcat agaaccctta agactttctc actagactgc agctgtgctg 720
   tgaaatactt aaaaacaaac actttttagt ttaaattcaa tttaatttaa aaatgcttat 780
   ttcatacaac gctttttaaa atgtatattt cgtagagttt tatcaagggg gcatttgcag 840
   gtagtctgaa attttcccct tgatatcact ataatttatt tcatttacac aatacacttt 900
   tatttagctt tttttttctt tttctttgag atggaatctc actctgtcac ccaggagtgc 960
   agttgcatga tctcggctca ctgcaccctc cgtctcccag gttcaagtga ttctcctgcc 1020
   ttagcctcct gattagctgg gattataggc acctaccatc acacccagct aatttatgta 1080
   tttttagtag agacagggtt tcaccatatt ggccagggtg gtctcgaact cctgacctca 1140
   agtgatccac ccttcttggc ctcccaaagt gctgggatta caggtatgag ctgcggatct 1200
   ggcctattta gcactttttg atgtggtagg aatacacaaa tggatgaggc atacttcctg 1260
   atcaaatagc ccattaatca tctggtatcc aaaaaaaaaa aggcccgaac aaaacaaaac 1320
   taaccgattc aaaataaacc ccggaaaaat ggaaaaaaat ttgtgatttt gtaaaaaant 1380
   gttcggaaag tgacantaaa actttcaggg gaggattggg gttagggaaa atttttgaca 1440
   catgggggga acccaggatt tatttaaaaa tttatttttg aaangggntc ccactgtcac 1500
   ccaggggggg gggnaaaacc aaaatcttag cccacggcca acctccaccc ccggggttaa 1560
   agcaattccc ctgcctcacc ccccgattac cggggattaa gggcacccac caccaaaccc 1620
   gacaaatttt tgttttttta aaaaaaaagc cagggggg                        1658
<210> 55
   <211> 3201
   <212> DNA
   <213> Homo sapiens
<400> 55
   caaaaacaca actaatcgaa tcaaaataaa actctgaaaa aatgataaat aaatagttgt 60
   gattttgtag ataagtgttc tgatagtgac agtaagacgt tcatgggagg attggtgtta 120
   gggaacatgt gtgacacatg aggtgaatcc aggatttatt taaatattta ttttttaaga 180
   cggagtctca ctgtcaccca ggctggagtg cagaagcaca attttagctc actgcaacct 240
   ttacctcctg ggttcaagcg attctcctgc gtcagccccc gagtagctgg gattacaggg 300
   acccaccaca cccgactaat ttttttgtat ttttaataga gacagggtct caccatgttg 360
   gccaggctgg tctngaactc ctgncctcaa atgatccacc ctcctcgaca tcccaaagtg 420
   ctgggattac aggcgtgagt cactgctcct ggcccaggat ttttttttta agagatgggg 480
   tcttgctatg ttgcccacgc ctaacagtct tgaactccta acagtcgatt ggcctcaaca 540
   atcctcctac ctcagcttct cgagtagctg ggactacagg catgttgtgc ccagcttaga 600
   tagtggaagg ttatttcagg acagaaagaa caacttgcat tagacatcat cagtatgatg 660
   aaagcatagg gttaagtggg gaggttaatt ggggggaatt gagacgggag aggagctagg 720
   agccaggtca tgaagggcct tatatgccat gaagaaaatt atggattgta acattctggt 780
   aatgaggtgt ccttataata cagcccattt taatagtcat taacaaagag aatctttgcc 840
   tttatcatcc attttcttat atttattcag gggcagattt attgaaatta actagagatg 900
   atgtgatcca aatctgtggc cctgcagatg gaatcagact ttttaatgca ttaaaaggcc 960
   ggtatgtaat tagtgttaaa atatcaataa ggttgggatt ccaaatggat ttggttcagg 1020
   aattcttcac aatactctac acagctaaat acaccttctc aaaacatttg ttttaaatgt 1080
   cagctatgta ccaaataaag attttagaaa aatgttttct tgcactttgt ccaaggaatt 1140
   ccccatgata ttccattttt catttccagg tgaatgaatt ttccaatgaa aattcatcat 1200
   ccttttcatg agccaggtac tattgtaggc tctgggaata ttaatatatt ggtgagcaaa 1260
   acagggtgcc cattgttagg gagtttactt tctaatgggg agtaagataa taaatattgt 1320
   ggatagtgat aagagctttg aaggaaaaca aaacaaataa gcaaaaaaca aggttgtggg 1380
   ctatatggtg cccagaatat ggtcaggaag ggcctctagg ggatacttaa gctgagttct 1440
   gagaaagatg cagctctgga gtcacaggaa gggagactgt tctaggcaga aggaacaaca 1500
   ggtgcaaagg tcccaagttg gttttaactg gagaagtggt cattcttaaa tgtgggtggg 1560
   aattactttt ttttaatttt tttgaaaaca gagtctcact ctttcgccca gactggagta 1620
   cagtggagca atcttggctc actgcaagct ccacctcccg ggttcacgcc attctcctgc 1680
   ctcagcctcc cgagtagctg ggactacagg cgcctgccac catgcccagc taattttttg 1740
   tattttttta gtagagacag ggtttcaccg tgttagccag gatggtctcg atctcctgat 1800
   ctcgtgatcc acccgccttg gcctcccaaa gtgctgggat tacaggcgtg agccaccgca 1860
   cccggcaaat gtgggtagga agtgtctgcc tgcaattgtt aaaatacata gtaatttgtg 1920
   gtcctttttt gggatttaca attttaaata ggtagtagta actgatgaaa aaagcattta 1980
   ggtcaatggc aacagaacta aatcagtgac tacatagtaa aacagagtgt gcagtattta 2040
   tttttggttt gcttttgttt tggggttttt ttcttcagac agggtcttgc tctgtcaccc 2100
   agcctggagt gcagcaatgc aatcatggct cactgcagcc tcgatttcct gggctcaagc 2160
   catcctcctg cctagatccc tgagcagctg ggactacagg cacgcaccac cacgcccgcc 2220
   taattttgta ccttttgtag aggcggggtt ttgccgtgtt cccctggctg gtctcaaact 2280
   cctgagctca agcaatcctt ctgactcgcc ttcctaaagt gctgggatta cagatgtgag 2340
   ccaccgtgcc tggtcatatt tgtttttgtc tacatcataa gggatgttga atcttggaaa 2400
   attttaagga aggaagtgaa atcaggggtt gatgacttgt ggaaaaataa ttgatctttc 2460
   attttttcaa gcatattatc taaagggtgt ttagaaattg gaaggagtaa gatgatcaga 2520
   tcaagaggta gttgaatacc tttctctaag aagaacagag aaaggactta ggctagttag 2580
   gagaaggtaa tgagagaaat aagatgatct agtttactgt gttggcattt tttagtaaaa 2640
   agtatatctt gaacctgacc cattagacgg tggcttttat attttgacta tttctaactt 2700
   ttacaccaaa ggccacaaac ccaaatgnct ttggggtaca tacaggtaat ntaagtgaat 2760
   ggccgtcaac actggggtat agtgaagaat atatgcctca tttaagaaga actaagattg 2820
   ctagatctgg gtcttcagga aaagccaaaa gttcagnggt ttatatgatg tctcatattg 2880
   gaactaattc cacttaagga aaaaaaaaac cctactactg gggaactaat aaaacatagc 2940
   aaattaattg catttggcca atgagcttcc aatttttcat ctgctctata cgattaatgt 3000
   agaaacacaa tttctccctt ctttttattg tgcttaccat aaagaatgta agattaatta 3060
   gccttttcct ccttaggatg gtgcgtccaa ggttaaccat ttatgtttgt caggaatcac 3120
   tgcagttgag ggagcagcaa caacagcagc agcaacagca gcagaagcat gaggatggag 3180
   actcaaatgg tactttcttc g                                          3201
<210> 56
   <211> 726
   <212> DNA
   <213> Homo sapiens
<400> 56
   taggacagat atgaagaaag gtgaagagat aacttatggg catgtgtgat gggaaactat 60
   caaacagcag atttagtatt tgctagaagt aacaataatc actagcattt attaagttcc 120
   taatacatgg caggtgccat tctttttttt tttttttttt tttgagacag tctcactcag 180
   ttacccaggc tgaagtacag tggcacaatc atgactcatt gcagccttga ccacctgggc 240
   tcaagtgatt ctctcccctc agcctaccga gtagctagaa ctaaagctac tcatttgcct 300
   aattgtttta tttattgtag agatgggatc tccctatgtt gcccaggctg gtcttgaact 360
   cctgggctca agcgatctcc ctgcctcagc ctcccaaatt gctgggacta taggtgtaaa 420
   ccactacacc cagccaggta ctattcttaa cactttttat atgctttaat atttacaact 480
   aatatctcct ctttgcggag gattttactg ttatctccgg tatttatgca agaaaaatca 540
   ggcctaggaa tgttaaataa cttgccctag gtcacacagc tgcaagtggt tgagccagga 600
   tttgaactta ggcagtgtga tttctgagtc cttcagaaaa gtgggcattc ctagtgatca 660
   agttacctgt aagagtggac aaactttgta ctgactgctg ttgctctctc ccttttgcag 720
   tttacc                                                            726
<210> 57
   <211> 393
   <212> DNA
   <213> Homo sapiens
<400> 57
   tctagtttac catgctatct atctagaaga actaacagct gttgaattga cagaaaaaat 60
   tgctcagctt ttcagcattt ccccttgcca gatcagccag atttacaagc aggggccaac 120
   aggaattcat gtgctcatca gtgatgaggt gagtttttca gtacagttga agtttaccag 180
   atgatgatta atttgtcagc gcatggagca agagcagttc tcctggggca gagtgttctg 240
   ttttctagat cagagggttt catacttttt tgcatgcttc ctcctaattt tttttttttt 300
   tgaaaaagnc ccccccggtg accaaggccg gattgaagtg gcccaaccca ggggagggna 360
   cccccccccc cgggttacat atgattcccc ggt                              393
<210> 58
   <211> 1487
   <212> DNA
   <213> Homo sapiens
<400> 58
   cctgggaggg tgaaactcat gcctttatct gggagaaggg agtttatccc tgggagaagg 60
   gagtttgaga agggaaagtg gggaaggaaa attaaagagg ttttaggaaa gagcacatat 120
   gggagttgaa gacacggaag tcagttctcc tgtatgcaca tatgaagaca cggaagtcag 180
   ttctcctgta tgcacatatt tgtgtccgtg tgctccttgg agagtcttgg ggttaaccca 240
   gggaaatgca taccaggctt taaaacaact cttccaatct gcttagtttt ttaatattta 300
   aaaaccaaat gggaggggag gaaaaaactt ttatgatcct agatattcaa gaaatacatt 360
   atttgtgacc tgtaaagcct cccatataaa tcccactgga tgtggggtag tccttaggga 420
   cacaggaata taaaggggtt gtttttcatt taaataatca caacatttag atgatacaga 480
   actttcagga agaagcatgt tttattctgg acacaatgaa aggtaatagt aagacaaaca 540
   tctttctcta agcagtttaa cagctttact actactaggc aaagtacata cttctttgag 600
   gagagatagg aaaagtaacc atatttgggt aaagaaagtg acaatattag agattgttcc 660
   tttttttaag ttggcttttt tttttttttt tttttttgaa ggaaaagtct cgatctgttg 720
   cccaggttgg agtgcagggc atgatctcgg gctcantaca accttcgctt cccaagttca 780
   agtgattctt cctccctctg cctcccaagt agctgggatt acaggcacgt gccaccatgt 840
   ctggctaatt tttgtatttt tagtagagat gggtttcacc ttgttggcca ggctggtctc 900
   gaactcctga cctcaagcga tccacccacc ttggcctccc aaagtgatgg gattacagac 960
   atgagccact gtgcctggcc cagctttttg ccattctctt ccatcttcca ttacagatga 1020
   ggaaattgag gcatgttgtt tttataatgc attttttttg agacagaatt tcgctcttgt 1080
   tgcccaagct ggagtccagt ggcgtgatct cagctcactg caacctccac ctcccttttc 1140
   tccccttctc acgagaatct taccatactt tttttctcct gctgtgtttt agaggtgact 1200
   aaagatgatt ctctctctct ctttttttta acaggaaacc aatgatagct atcatatcat 1260
   actgaagtag gagtgcggcg tttcgtgccc agtggctgct ccttccttca cctctgaaaa 1320
   cggccctctt gaagggggat atgaatggag atttgaaggt ctgcaagaac ctgactcgtc 1380
   tgactgtgtg tggaggagtc caggccatgg aggcagaatc ctgccctctg tgttggccca 1440
   agctcttgtg gtacacacag agggccagga ttctgcctcc atggcct              1487